# EUROPEAN PATENT APPLICATION

(11) **EP 4 834 285 A1**
(43) Date of publication of application: **04.11.2026**
(21) Application number: 24911425.7
(22) Date of filing: 27.12.2024
(51) Int. Cl.: A61F 2/24

(54) **DELIVERY SYSTEM FOR ARTIFICIAL IMPLANT**

(30) Priority: 29.12.2023 CN 202311869046
(71) Applicant: Venus MedTech (HangZhou), Inc., Hangzhou, Zhejiang 310000 (CN)
(72) Inventor: GONG, Quangang, Hangzhou, Zhejiang 310000 (CN); ZHANG, Zhifei, Hangzhou, Zhejiang 310000 (CN); MA, Renzheng, Hangzhou, Zhejiang 310000 (CN); LI, Yaru, Hangzhou, Zhejiang 310000 (CN); RUI, Dan, Hangzhou, Zhejiang 310000 (CN); ZHANG, Heqiang, Hangzhou, Zhejiang 310000 (CN); LIM, Hou-Sen, Hangzhou, Zhejiang 310000 (CN); ZENG, Xiaotong, Hangzhou, Zhejiang 310000 (CN); ZHAN, Xiaodong, Hangzhou, Zhejiang 310000 (CN); WU, Wenwen, Hangzhou, Zhejiang 310000 (CN)
(74) Representative: KIPA AB
(86) International application number: PCT/CN2024/143052
(87) International publication number: WO 2025/140505

(57) **Abstract**

A delivery system for an artificial implant comprises a control handle (400) and a catheter assembly (200) having a proximal end connected to the control handle. The catheter assembly (200) includes an outer sheath (240) and an inner shaft assembly positioned within the outer sheath (240). The artificial implant (100) is connected to a distal end of the inner shaft assembly and is controlled by the control handle (400). The control handle (400) includes a first handle **(41)** and a second handle (42) cooperating with each other, wherein the first handle **(41)** is configured to drive axial movement of the outer sheath (240) to effect deployment and retrieval of the artificial implant, and the second handle (42) is configured to control release of the artificial implant to detach the artificial implant from the delivery system.

## Description

### TECHNICAL FIELD

The present disclosure relates to the technical field of medical devices, and in particular, to a delivery system for an artificial implant.

### BACKGROUND ART

Percutaneous prosthetic heart valve replacement and repair represent an advanced interventional treatment technique for heart valves, which allows repair or replacement of a heart valve without open-chest surgery via an interventional approach.

Percutaneous implantation techniques can achieve less invasiveness than surgical procedures such as open-heart surgery. In percutaneous implantation techniques, a loaded implant or prosthetic valve is mounted on a distal end portion of a flexible catheter and advanced through a patient's blood vessel or through a percutaneous puncture until the prosthetic valve reaches the implantation site. The prosthetic valve at the distal end of the catheter is then expanded to its functional size at the site of the diseased native valve.

In interventional implantation procedures for prosthetic valves, the following problems often exist:
First, when a self-expanding prosthetic valve reaches the implantation site in the body, the operator retracts the delivery sheath to release the prosthetic valve. At this time, the prosthetic valve tends to "jump" out very quickly from the distal end of the sheath. In other words, the outward biasing force of the prosthetic valve's frame tends to cause the prosthetic valve to be ejected very quickly from the distal end of the delivery sheath, making it difficult to deliver the prosthetic valve from the sheath in a precise and controlled manner and increasing the risk of trauma to the patient.

Second, in existing prosthetic valve products, the valve is typically recaptured or retrieved by advancing the sheath to crimp the valve when the valve is two-thirds expanded. However, when the valve is fully expanded, i.e., completely detached from the sheath, it is often very difficult to retrieve the prosthetic valve.

The present disclosure provides a delivery system that enables retrieval of an artificial implant even when the artificial implant is fully deployed/expanded.

The present disclosure provides a delivery system for an artificial implant, having opposite distal and proximal ends, the delivery system comprising a control handle and a catheter assembly having a proximal end connected to the control handle, wherein:
the control handle comprises a first handle and a second handle cooperating with each other;
the catheter assembly comprises an outer sheath and an inner shaft assembly located within the outer sheath, the artificial implant being connected to a distal end of the inner shaft assembly and being controlled by the control handle, the first handle being configured to drive axial movement of the outer sheath to effect deployment and collapse of the artificial implant; the second handle being configured to control release of the artificial implant to detach the artificial implant from the delivery system.

When the artificial implant is fully deployed/expanded (i.e., in its implanted configuration), the artificial implant is no longer constrained by the outer sheath. The present invention allows, via the second handle, further compression of the artificial implant in the fully expanded state, for example by compressing or folding a proximal portion of the artificial implant, and then advancing the outer sheath distally to retrieve the artificial implant. Through cooperation between the first handle and the second handle, the artificial implant can be quickly retrieved back into the catheter system.

Several optional arrangements are also provided below. They are not intended to impose additional limitations on the above general solution, but rather to serve as further supplements or preferences. Provided there is no technical or logical contradiction, the optional arrangements may be combined with the above general solution individually, or multiple optional arrangements may be combined with one another.

Optionally, each of the handles comprises a support member and a drive mechanism disposed on the support member.

The support member of the first handle comprises a first support body and a support rod fixed to the first support body and extending further proximally. The support member of the second handle comprises a second support body slidably mounted on the support rod. A sliding locking mechanism is further provided on a distal side of the second support body itself, the sliding locking mechanism interacting with the support rod to maintain a relative position with respect to the first support body.

Optionally, the catheter assembly comprises, from outside to inside, an outer sheath and at least one shaft, specifically:
an outer sheath having a proximal end movably connected to a first base of the first handle, and a distal end configured to cover the artificial implant;
a third shaft having a proximal end fixed to the second handle;
a second shaft having a proximal end movably connected to a second base of the second handle;
a first shaft having a proximal end movably connected to a proximal portion of the second handle.

Optionally, a first extension sleeve is further fixed to a distal end of the first support body, and the catheter assembly further comprises:
a jacket disposed externally of the outer sheath, the jacket having a proximal end fixed to the first extension sleeve.

Optionally, a first support base is fixed within the first extension sleeve, the proximal end of the jacket is fixed to the first support base, the outer sheath sealingly extends through the first support base, and the first support base is provided with a first venting structure.

Optionally, a second support base is fixed to a distal portion of the second support body, the proximal end of the third shaft is fixed to the second support base, the second shaft sealingly extends through the second support base, and the second handle is provided with a second venting structure.

Optionally, the first venting structure and the second venting structure are one-way valves.

Optionally, a second extension sleeve is further fixed to a proximal end of the second support body, and the drive mechanism comprises:
a first drive mechanism disposed on the first support body and operatively coupled to the outer sheath;
a second drive mechanism disposed on the second support body and operatively coupled to the second shaft;
a third drive mechanism disposed on the second extension sleeve and operatively coupled to the first shaft.

Optionally, the third drive mechanism comprises a third base slidably mounted within the second extension sleeve, and a third drive sleeve threadedly engaged with the third base and rotatably mounted on the second extension sleeve.

Optionally, the second extension sleeve is provided with a third support base, the second venting structure is disposed on the third support base, and a vent tube extending through the second support body is provided between the third support base and the second support base.

Optionally, the distal portion of the second support body comprises a partition plate disposed along the axial direction, the partition plate being provided with a through-hole for passage of the vent tube, and the second support body is provided with a groove serving as a passage.

Optionally, a lock base is connected to a distal end of the third shaft, the lock base being provided with a locking hole or a groove;
a locking wire is connected to a distal portion of the second shaft, the locking wire being configured to be threaded through the artificial implant and then connected to the lock base to constrain or release the artificial implant;
a locking rod or a circumferentially arranged protruding structure is connected to a distal end of the first shaft, for maintaining the connection between the locking wire and the lock base.

Optionally, one end of the locking wire is a drive end connected to the second shaft, and the other end of the locking wire is a working end, the working end being configured to be threaded through the artificial implant and then tightened or loosened relative to the lock base.

Optionally, the locking wire is provided with a radiopaque marker.

Optionally, a distal end of the locking wire is provided with a radiopaque marker.

Optionally, the radiopaque marker is connected to the locking wire by bonding, sewing, braiding, crimping, or other processes.

The control handle comprises a rotating member sleeved on the support body, the rotating member having a tubular configuration with opposite inner and outer walls. The rotational locking mechanism comprises:
a first tubular member fixed to the support body, wherein the control handle has an installation channel extending axially through the support body, a portion of the installation channel extending through the interior of the first tubular member;
a second tubular member rotatably sleeved on an outer periphery of the first tubular member, a portion of the second tubular member being a working section extending into the rotating member, the working section having a protrusion on an outer wall thereof;
a locking member positioned within a radial gap between the working section and the rotating member, and configured to be slidable radially relative to the rotating member and to act against an inner wall of the rotating member, wherein upon rotation of the second tubular member, the protrusion presses against the locking member to move the locking member and thereby lock the rotating member;
an operating member configured to drive rotation of the second tubular member.

Optionally, the side walls of the support body and the first tubular member are locally open on the same radial side to form an installation opening.

Optionally, a side wall of the second tubular member is open on one radial side, and the open position is circumferentially offset from the open position of the first tubular member.

Optionally, the support body comprises:
a main body being cylindrical and extending axially along the control handle, the first tubular member being located on a proximal side of the main body;
a connecting seat fixed to a proximal end of the main body, a radial gap being left between the connecting seat and the first tubular member, the second tubular member being radially positioned within the radial gap.

Optionally, the connecting seat is a housing structure having a semi-cylindrical surface, and the support body further comprises a half-shell that engages with the connecting seat to form a cylindrical shape, the half-shell being provided with a clearance window, the operating member being exposed through the clearance window.

Optionally, the connecting seat is provided with a guide slot extending radially therethrough, the locking member comprises a first locking member slidably disposed within the guide slot, and the protrusion on the second tubular member abuts against a radially inner side of the first locking member.

Optionally, a wall of the guide slot is provided with a registration structure that cooperates with the first locking member.

Optionally, the locking member comprises:
a first locking member slidably fitted in the connecting seat;
a second locking member radially movably engaged with the connecting seat in a snap-fit manner, the first locking member and the second locking member moving in opposite directions synchronously.

Optionally, the second locking member is semi-annular and is provided with cooperating guide structures with the support body.

Optionally, the connecting seat has a stop portion that abuts against the operating member to limit a rotational range thereof.

Optionally, the first locking member and the second locking member are at the same axial position and are located on radially opposite sides of the second tubular member.

Optionally, a plurality of locking members are arranged along the axial direction.

Optionally, a stop member is embedded at a position where the locking member cooperates with the rotating member.

Optionally, the first locking member and the second locking member are formed of a hard material, and the stop member is formed of a soft material.

Optionally, the rotating member is a drive sleeve rotatably sleeved on an outer periphery of the support body.

Optionally, the height of an outer surface of the protrusion gradually increases along a circumferential direction of the second tubular member.

Optionally, the second tubular member has opposite positions:
a locked position, in which the protrusion is pressed against the locking member, thereby acting on the rotating member;
a released position, in which the protrusion releases the pressing force against the locking member;
the locking member having an arcuate surface or radially undulating structure that cooperates with the protrusion.

Optionally, two support rods are arranged side by side, with positioning teeth arranged on opposing sides thereof;
the second support body is provided with guide holes, the two support rods extending into respective guide holes, the sliding locking mechanism cooperating with the positioning teeth to maintain the relative position between the second handle and the first support body.

Optionally, the two support rods are slidably fitted with the guide holes.

Optionally, the support rod is a metal strip having a strip-shaped cross-section, and the extension direction of the metal strip is perpendicular to the spacing direction of the two support rods.

Optionally, the sliding locking mechanism comprises:
a locking member movably mounted on the second support body, having a locked position engaged with the positioning teeth and an unlocked position disengaged from the positioning teeth;
an elastic member acting between the locking member and the second support body, biasing the locking member toward the locked position;
an operating button acting on the locking member, driving the locking member toward the unlocked position.

Optionally, the locking member is provided with a mounting hole and is slidably sleeved on the support rod through the mounting hole, a clearance being provided between the mounting hole and the support rod in a direction perpendicular to the sliding direction of the support rod, and the locking member moving within the clearance when moving between the locked and unlocked positions.

Optionally, one side of the mounting hole faces the positioning teeth, and the side is provided with an engaging portion that cooperates with the positioning teeth.

Optionally, the engaging portion comprises one or more snap-fit teeth arranged at intervals along the sliding direction of the locking member.

Optionally, two sliding locking mechanisms are provided, each acting on a respective support rod, and the operating buttons thereof are radially aligned with each other along a radial direction of the second handle and are arranged in opposite directions.

Optionally, an abutment rib is fixed within the second support body, and the elastic member is compressed and acts between the abutment rib and the locking member.

Optionally, the abutment ribs of the two sliding locking mechanisms are spaced apart from each other, with an installation channel extending through the spacing.

Optionally, the locking member is provided with a positioning seat on a side facing the elastic member, and the elastic member is inserted into or sleeved onto the positioning seat.

Optionally, the elastic member is a spring.

Optionally, the distal portion of the second support body is a second connecting seat comprising two partition plates and a transition portion connected between the two partition plates;
the transition portion is a housing structure having a semi-cylindrical surface, the second support body further comprising a second half-shell that engages with the transition portion to form a cylindrical shape, the sliding locking mechanism being positioned in a region enclosed by the transition portion and the second half-shell, and only a portion of the operating button being exposed.

Optionally, the second support body further comprises a snap-fit seat engaged between the second connecting seat and the second half-shell, the snap-fit seat having an open slot through which the operating button is exposed.

Optionally, the open slot is circumferentially closed and extends along the direction of movement of the operating button.

Optionally, there are two support rods, and two connecting sleeves are fixed to a proximal portion of the first support body, distal portions of the two support rods being fixedly inserted into the respective connecting sleeves.

The distal portion of the second support body comprises two partition plates arranged at axial intervals, each partition plate being provided with a guide hole, the second support body being provided on radially opposite sides with axially extending third slide grooves, each support rod passing through a corresponding guide hole and being received in a corresponding third slide groove, the second handle as a whole being slidably fitted along the two support rods relative to the first handle.

Optionally, the second handle has two extreme positions along the two support rods relative to the first handle: a first extreme position where the second handle abuts against the first handle, and a second extreme position where the second handle is spaced apart from the first handle.

Optionally, the control handle has an installation channel extending axially through the first support body and the second support body, the first support body comprising:
a first main body being cylindrical and extending axially along the control handle;
a first connecting seat fixed to a proximal end of the first main body, the two connecting sleeves being fixed to the first connecting seat and located on radially opposite sides of the installation channel.

Optionally, a side wall of the connecting sleeve and a distal end of the support rod are provided with positioning holes at corresponding positions, and are fixedly connected by a connector extending through the positioning holes.

Optionally, the first connecting seat is a housing structure having a semi-cylindrical surface, the first support body further comprising a first half-shell that engages with the first connecting seat to form a cylindrical shape.

Optionally, the connecting sleeves are positioned at an interface between the first connecting seat and the first half-shell, and an outer wall of the connecting sleeve is provided with a snap-fit that cooperates with the first half-shell.

Optionally, a portion of the first main body is radially open to form a first guide slot, and the drive mechanism comprises:
a first base slidably disposed in the first guide slot, the first base being configured to be connected to a controlled member;
a first drive sleeve rotatably sleeved on an outer periphery of the first support body, an inner wall of the first drive sleeve being threadedly engaged with the first base.

Optionally, the control handle has an installation channel extending axially through the first support body and the second support body, the second support body comprising:
a second main body being cylindrical and extending axially along the control handle, the third slide grooves being located on a side of the second main body opposite the installation channel;
a second connecting seat comprising the two partition plates and a transition portion connected between the two partition plates, one of the partition plates being fixed to a distal end of the second main body;
a third connecting seat fixed to a proximal end of the second main body.

Optionally, the two partition plates comprise a first partition plate at the distal end and a second partition plate at the proximal end, a cover plate being engaged on a distal side of the first partition plate and defining a clearance region corresponding in position to the installation channel and the guide holes.

Optionally, the distal side of the first partition plate is provided with a positioning rib extending radially, and the cover plate is provided with a positioning groove that cooperates with the positioning rib.

Optionally, the two partition plates each extend radially open from their respective installation channel positions to their respective edges in a first radial direction;
the cover plate extends radially open from its installation channel position to its edge in a second radial direction opposite the first radial direction, thereby forming the positioning groove.

Optionally, a proximal portion of the third slide groove is a terminal section extending to an outer periphery of the third connecting seat, and a cooperating snap-fit structure is provided between a groove wall of the terminal section and the support rod.

Optionally, the snap-fit structure comprises:
an engagement block protruding from the groove wall of the third slide groove;
a snap-fit groove provided on the support rod and cooperating with the engagement block.

Optionally, when the second handle slides to abut against the first handle, the engagement block fits into the snap-fit groove.

Optionally, an anti-detachment member is provided at a proximal portion of the support rod, and when the second handle slides proximally relative to the first handle to an extreme position, the anti-detachment member is stopped by the partition plate.

Optionally, a portion of the second main body extends radially therethrough to form a second guide slot, and the drive mechanism comprises:
a second base slidably disposed in the second guide slot, the second base being configured to be connected to a controlled member;
a second drive sleeve rotatably sleeved on an outer periphery of the second support body, an inner wall of the second drive sleeve being threadedly engaged with the second base.

Optionally, the transition portion is a housing structure having a semi-cylindrical surface, and the second support body further comprises a second half-shell that engages with the transition portion to form a cylindrical shape.

Optionally, the artificial implant comprises:
a stent having a tubular shape and a mesh structure, the interior of the stent defining a blood flow passage;
a plurality of leaflets disposed within the blood flow passage and cooperating with each other to control blood flow therethrough, each leaflet having a fixed edge secured to the stent and a free edge cooperating with the other leaflets.

Optionally, the artificial implant further comprises an inner skirt positioned within the blood flow passage and connected to an inner surface of the stent, and a plurality of sealing members connected to the inner skirt, the sealing members being embedded in and protruding outwardly from corresponding mesh structures.

The present disclosure also provides a delivery system for an artificial implant, having opposite distal and proximal ends, the delivery system comprising a control handle and a catheter assembly having a proximal end connected to the control handle, the artificial implant being connected to a distal end of the catheter assembly and being controlled by the control handle, wherein the control handle comprises a support member and a drive mechanism disposed on the support member;
the support body being provided with a guide slot extending axially, the drive mechanism comprising a base movable along the guide slot and a drive sleeve rotatably mounted on an outer periphery of the corresponding support body, the drive sleeve being threadedly engaged with the base, and the support body being provided, on a proximal side of its guide slot, with a rotational locking mechanism configured to restrict or permit rotation of the drive sleeve.

The present invention also discloses a control handle having opposite distal and proximal ends, the control handle comprising a first handle and a second handle cooperating with each other; each of the handles comprising a support member and a drive mechanism disposed on the support member, the support member of the first handle comprising a first support body and a support rod fixed to the first support body and extending further proximally, the support member of the second handle comprising a second support body slidably mounted on the support rod, a sliding locking mechanism being further provided on a distal side of the second support body itself, the sliding locking mechanism interacting with the support rod to maintain a relative position with respect to the first support body.

The present invention also discloses a delivery system for an artificial implant, having opposite distal and proximal ends, the delivery system comprising a control handle and a catheter assembly having a proximal end connected to the control handle, the control handle comprising a first handle and a second handle cooperating with each other; the catheter assembly comprising an outer sheath and an inner shaft assembly located within the outer sheath, the artificial implant being connected to a distal end of the inner shaft assembly and being controlled by the control handle, the first handle being configured to drive axial movement of the outer sheath to effect deployment and collapse of the artificial implant; the second handle being configured to control release of the artificial implant to detach the artificial implant from the delivery system.

Optionally, the artificial implant is connected to a distal end of the catheter assembly of the delivery system via a wire.

Optionally, a wire control mechanism is provided at a distal end of the catheter assembly, the wire control mechanism comprising a lock base, a locking wire, and a protruding structure cooperating with the lock base.

Optionally, a wire control mechanism is provided on the second handle or distally on the catheter assembly, the wire control mechanism comprising at least one of a mechanism for clamping the wire or a mechanism for releasing the wire.

Optionally, the second handle has a first state in which it slides away from the first handle, and a second state in which it is connected to the first handle. An axially extending support rod is provided between the first handle and the second handle, wherein one of the first handle and the second handle is provided with the support rod while the other is provided with a guide hole, such that when the second handle moves relative to the first handle, the support rod moves axially relative to the guide hole.

Optionally, a first locking mechanism is further provided on a distal side of the second support body itself, the first locking mechanism interacting with the support rod to maintain a relative position with respect to the first support body.

Optionally, the support member of the first handle comprises a first support body and a support rod fixed to the first support body and extending further proximally, the support member of the second handle comprises a second support body slidably mounted on the support rod, a first locking mechanism being further provided on a distal side of the second support body itself, the first locking mechanism interacting with the support rod to maintain a relative position with respect to the first support body.

Optionally, a second locking mechanism is disposed in the first handle and/or the second handle for controlling movement thereof.

The present invention also provides a delivery system for an artificial implant. The delivery system has opposite distal and proximal ends and comprises a control handle and a catheter assembly, the catheter assembly having a proximal end connected to the control handle. The artificial implant is connected to a distal end of the catheter assembly and is controlled by the control handle. The control handle comprises a first handle and a second handle cooperating with each other. Each of the handles comprises a support member and a drive mechanism disposed on the support member. The support member of the first handle comprises a first support body and a support rod fixed to the first support body and extending further proximally. The support member of the second handle comprises a second support body slidably mounted on the support rod. Each support body is provided with a guide slot extending axially. The drive mechanism comprises a base movable along the guide slot and a drive sleeve rotatably mounted on an outer periphery of the corresponding support body, and the drive sleeve is threadedly engaged with the base. Each support body is provided, on a proximal side of its guide slot, with a rotational locking mechanism configured to restrict or permit rotation of the drive sleeve.

Optionally, a sliding locking mechanism is further provided on a distal side of the second support body itself, the sliding locking mechanism interacting with the support rod to maintain a relative position with respect to the first support body.

The present invention also discloses a delivery system for an artificial implant, having opposite distal and proximal ends, the delivery system comprising a control handle and a catheter assembly having a proximal end connected to the control handle, the control handle comprising a first handle and a second handle cooperating with each other; the catheter assembly comprising an outer sheath and an inner shaft assembly located within the outer sheath, the artificial implant being connected to a distal end of the inner shaft assembly and being controlled by the control handle, the first handle being configured to drive axial movement of the outer sheath to effect deployment and collapse of the artificial implant; the inner shaft assembly comprising a steering member, and the second handle comprising a steering drive mechanism configured to drive movement of the steering member.

The present invention also discloses a wire control mechanism for an artificial implant, having opposite distal and proximal ends, the wire control mechanism comprising: a lock base comprising a guide portion, a reduced-diameter portion, and a connecting portion in that order, wherein the connecting portion is provided with a locking hole and a wire hole, respectively, the guide portion is provided with a guide hole in a position corresponding to the locking hole, the reduced-diameter portion defines a radially open engagement region on its outer periphery, a distal end of the connecting portion tapers to form, on an outer periphery thereof, an expansion region that communicates with the engagement region; a locking rod slidably fitted with the lock base and inserted into the locking hole via the guide hole; a locking wire having a drive end at one end extending proximally through the wire hole, and a working end at the opposite end configured to be threaded through the artificial implant and then coupled to the locking rod.

In the delivery system of the present disclosure, the support rod in the handle serves as a connecting member between the second handle and the first handle to enhance the structural strength of the control handle, and cooperates with the sliding locking mechanism to maintain the length dimension of the control handle and meet operational requirements, such as increasing the speed of retrieving the artificial implant, or allowing rapid retraction of the distal structure after successful release of the artificial implant.

### BRIEF DESCRIPTION OF THE DRAWINGS

FIG. 1 is a structural view of a delivery system according to an embodiment of the present disclosure.
FIG. 2 is a structural view of the control handle of FIG. 1.
FIG. 3 is a structural schematic view of the control handle of FIG. 1, showing the second handle sliding proximally relative to the first handle.
FIG. 4 is a cross-sectional view of the control handle of FIG. 2 taken along its axis.
FIG. 5 is an exploded view of the second handle of the control handle according to the present disclosure.
FIG. 6 is an enlarged view of portion A of FIG. 1.
FIG. 7 is a partial structural view of the wire control mechanism according to an embodiment of the present disclosure, showing the locking rod engaged with the lock base.
FIG. 8 is a cross-sectional view of a catheter assembly according to an embodiment of the present disclosure.
FIG. 9 is a schematic view of the distal end of the delivery system according to an embodiment of the present disclosure, showing a state where the artificial implant is released and the locking wire is loosened.
FIG. 10 is a schematic view of the distal end of the delivery system according to an embodiment of the present disclosure, showing a state where the artificial implant is released and the locking wire is detached.
FIG. 11 is a structural view of the second support body of the control handle according to an embodiment of the present disclosure.
FIG. 12 is an exploded view of the support rod and the first handle of the control handle according to an embodiment of the present disclosure.
FIG. 13 is an exploded view of the sliding locking mechanism of the control handle according to an embodiment of the present disclosure.
FIG. 14 is an enlarged view of portion B of FIG. 4 (with the sliding locking mechanism in a locked state).
FIG. 15 is an enlarged view of portion B of FIG. 4 (with the sliding locking mechanism in an unlocked state).
FIG. 16 is a structural view of the locking member of the control handle according to an embodiment of the present disclosure.
FIG. 17 is a schematic view of the assembly of the locking member and the support rod of the control handle according to an embodiment of the present disclosure.
FIG. 18 is a structural view of the operating button of the control handle according to an embodiment of the present disclosure.
FIG. 19 is an exploded view of the second half-shell and the second drive sleeve of the control handle according to an embodiment of the present disclosure.
FIG. 20 is an exploded view of the operating button of the control handle according to an embodiment of the present disclosure.
FIG. 21 is a structural view of the snap-fit seat of the control handle according to an embodiment of the present disclosure.
FIG. 22 is an exploded view of the control handle of FIG. 2, showing the rotating member and the support body.
FIG. 23 is an exploded view of the locking member and the half-shell of FIG. 22.
FIG. 24 is a structural view of the support body of FIG. 23.
FIG. 25 is a front view of a second tubular member according to an embodiment of the present disclosure.
FIG. 26 is a structural view of the second tubular member of FIG. 25.
FIG. 27 is a structural view of the second tubular member of FIG. 26 from another perspective.
FIG. 28 is a front view of a control handle according to an embodiment of the present disclosure.
FIG. 29 is a cross-sectional view of FIG. 28 taken along line F-F (with the locking device in an unlocked state).
FIG. 30 is a cross-sectional view of FIG. 28 taken along line F-F (with the locking device in a locked state).
FIG. 31 is a structural view of a locking member (first locking member) according to an embodiment of the present disclosure.
FIG. 32 is an exploded view of the first locking member and the support body of FIG. 31.
FIG. 33 is an enlarged view of portion C of FIG. 22.
FIG. 34 is an exploded view of the second locking member and the support body of FIG. 33.
FIG. 35 is an enlarged view of portion D of FIG. 24.
FIG. 36 is a cross-sectional view of FIG. 28 taken along line E-E (with the locking device in an unlocked state).
FIG. 37 is a cross-sectional view of FIG. 28 taken along line E-E (with the locking device in a locked state).
FIG. 38 is a partial structural view of the control handle according to an embodiment of the present disclosure, showing the second handle in the second extreme position.
FIG. 39 is a structural view of the first half-shell of the control handle according to an embodiment of the present disclosure.
FIG. 40 is a structural view of the snap-fit structure between the support rod and the slide groove of the control handle according to an embodiment of the present disclosure.
FIG. 41 is a partial structural view of the proximal portion of the second support body of FIG. 11.
FIG. 42 is an enlarged view of portion G of FIG. 38.
FIG. 43 is a partial structural view of the support rod and the anti-detachment member of FIG. 12.
FIG. 44 is a structural view of an artificial implant according to an embodiment of the present disclosure.
FIG. 45 is a partial view of FIG. 44, showing the sealing member.
FIG. 46 is a structural schematic view of a lock base according to the present disclosure.
FIG. 47 is a schematic view of a lock base according to the present disclosure.
FIG. 48 is a schematic view of FIG. 47 taken from direction E.
FIG. 49 is a schematic cross-sectional view of the lock base of FIG. 48 taken along line G-G.
FIG. 50 is a schematic view of the connection between an artificial implant and a control wire according to an embodiment of the present disclosure.
FIG. 51 is a schematic view of the control wire of FIG. 50, showing the second end thereof detached from the lock base.
FIG. 52 is an enlarged view of portion H of FIG. 50.
FIG. 53 is an enlarged view of the retaining member of FIG. 52, showing the clearance for the movement path of the anti-detachment structure.
FIG. 54 is a structural schematic view of the anti-detachment structure.
The reference numerals in the figures are set forth as follows:
100, artificial implant; 110, stent; 111, mesh structure; 112, inflow side; 120, leaflet; 130, sealing member; 140, inner skirt; 150, blood flow passage;
200. catheter assembly; 210, inner core; 220, wire control tube; 230, inner sheath tube; 240, outer sheath; 250, jacket;
31, lock base; 311, guide portion;
32, locking rod; 312, reduced-diameter portion; 313, connecting portion; 314, wire hole; 315, locking hole; 316, guide hole; 317, engagement region; 318, expansion region;
33, locking wire; 331, radiopaque marker; 34, control wire; 341, first end; 342, second end; 343, anti-detachment structure; 35, lock base; 351, engagement groove;
400, control handle; 401, proximal end; 402, distal end; 403, installation channel; 404, first viewing window; 405, second viewing window;
41, first handle; 410, first support body; 4101, second slide groove; 4102, first guide slot;
411, first main body; 412, first connecting seat; 4121, guide slot; 4122, positioning groove;
4123, first slide groove; 4124, stop portion; 414, first half-shell; 415, snap-fit; 416, limiting rib;
418, first extension sleeve; 4181, clearance window; 419. first support base; 4191, first venting structure;
42, second handle; 420, second support body; 4201, abutment rib; 4202, spacing region; 4203, clamping plate; 4204, mounting region
421. second main body; 4211, third slide groove;
422, second connecting seat; 4212, second guide slot; 4213, groove; 4221, partition plate;
4221a, first partition plate; 4221b, second partition plate; 4222, guide hole; 4223, transition portion; 4225, positioning rib; 4226, relief hole;
423, third connecting seat; 4231, engagement block;
424, cover plate; 4241, clearance region; 4242, positioning groove;
425, second half-shell; 426, snap-fit seat; 4261, open slot;
427, second extension sleeve; 428, second support base; 429, third support base; 4291, second venting structure;
430, sliding locking mechanism; 431, locking member; 4311, mounting hole; 4312, clearance; 4313, engaging portion; 4314, snap hole; 4315, positioning seat;
432, elastic member; 433, operating button; 4331, snap post;
440, connecting sleeve; 441, positioning hole; 442, connector;
450, support rod; 4501, snap-fit groove; 4502, positioning tooth;
451, anti-detachment member; 4511, nut; 4512, screw rod; 452, support strip; 453, rack; 4521, mounting groove;
460, rotational locking mechanism; 461, first tubular member; 4611, installation opening;
462, second tubular member; 4621, working section; 4622, protrusion; 4623, opening;
463, locking member; 4631, first locking member; 4632, positioning block; 4633, first sliding portion; 4635, second locking member; 4637, relief region; 4638, arcuate surface; 4639, second sliding portion;
464, operating member; 4641, anti-slip portion; 4642, indicator; 4643, stop member;
470, vent tube; 480, rotating member; 481, first drive sleeve; 482, second drive sleeve; 483, third drive sleeve
491, first base; 492, second base; 493, third base.

### DETAILED DESCRIPTION

The technical solutions of the embodiments of the present disclosure will be described clearly and completely below with reference to the accompanying drawings of the embodiments of the present disclosure. It is apparent that the described embodiments are only a part of, and not all of, the embodiments of the present disclosure. Based on the embodiments of the present disclosure, all other embodiments obtained by those of ordinary skill in the art without creative efforts shall fall within the scope of protection of the present disclosure.

It should be noted that when a component is referred to as being "connected" to another component, it may be directly connected to the other component or an intervening component may also be present. When a component is considered to be "disposed on" another component, it may be directly disposed on the other component or an intervening component may also be present.

Unless otherwise defined, all technical and scientific terms used herein have the same meaning as commonly understood by one of ordinary skill in the art to which this disclosure belongs. The terminology used herein in the description of the disclosure is for the purpose of describing particular embodiments only and is not intended to limit the disclosure. As used herein, the term "and/or" includes any and all combinations of one or more of the associated listed items.

In this disclosure, the terms "first", "second", etc. are used for descriptive purposes only and are not to be construed as indicating or implying relative importance or implicitly indicating the number or order of the technical features indicated. Thus, features defined as "first" or "second" may explicitly or implicitly include one or more of such features. In the description of this disclosure, "plurality" means at least two, for example, two, three, etc., unless otherwise specifically and expressly limited.

This specification describes an artificial implant and a delivery system for delivering the artificial implant into a subject's body. The delivery system comprises a control handle and a catheter assembly. The control handle is connectable to and controls the catheter assembly for performing an interventional procedure. The catheter assembly comprises a plurality of controlled members, the distal ends of which cooperate with each other to operate the artificial implant, for example, to release, retrieve, lock position, steer, adjust spatial orientation, and the like. Each controlled member may be a hollow tube, a solid rod, a flexible wire, or a combination thereof. There are a plurality of controlled members, and at least two of them (taking the proximal end as an example) can slide axially relative to each other or rotate relative to each other about the axis.

The artificial implant may be a prosthetic heart valve or a vascular stent, etc. The prosthetic heart valve may comprise a stent and leaflets connected to the stent for controlling the flow of blood. The number of leaflets is plural, typically two or three. Skirt designs, such as the sealing member described below, may be added to the inner and/or outer side of the stent as needed. The stent itself may be formed by braiding or tube cutting. The prosthetic heart valve may be used to replace a diseased valve in the heart, such as the aortic valve.

Hereinafter, when used to indicate direction, "proximal" generally refers to the side adjacent to the operator (e.g., a physician), and "distal" refers to the opposite side farther away. Along the interventional path, each component has its own opposite distal and proximal ends. Theoretically, when the catheter assembly and the control handle are fully straightened, the line between the proximal end and the distal end defines the axial direction, thereby also defining the radial direction perpendicular to the axial direction and the circumferential direction arranged around the axis. When used to refer to a structure, "end" in the text indicates the endpoint of the structure, or a point, region, or specific structure connected at that point or region on that side.

The present disclosure provides a delivery system for an artificial implant, having opposite distal and proximal ends. The delivery system comprises a control handle and a catheter assembly 200 having a proximal end connected to the control handle 400. The control handle 400 comprises a first handle 41 and a second handle 42 cooperating with each other. The catheter assembly 200 comprises an outer sheath 240 and an inner shaft assembly located within the outer sheath 240. The artificial implant 100 is connected to a distal end of the inner shaft assembly and is controlled by the control handle 400. The first handle 41 is configured to drive axial movement of the outer sheath 240 to effect deployment and collapse of the artificial implant 100. The second handle 42 is configured to control release of the artificial implant 100 to detach the artificial implant 100 from the delivery system.

In the present disclosure, the artificial implant may be a prosthetic heart valve, an occluder, or a cardiac repair device, such as a mitral or tricuspid clip for heart valve repair.

The outer sheath 240 moves axially relative to the artificial implant 100. During interventional delivery, the outer sheath 240 covers the artificial implant 100, radially constraining the artificial implant 100 and limiting its radial expansion. When the outer sheath 240 is moved to expose part or all of the artificial implant 100, the exposed portion expands. When retrieval is required, moving the outer sheath 240 in the opposite direction re-covers the artificial implant 100, thereby radially collapsing and folding the artificial implant 100.

The axial movement of the outer sheath 240 relative to the artificial implant 100 determines whether the artificial implant 100 is exposed. However, it does not strictly require that the exposed portion immediately expands radially; at least the radial constraint is removed, allowing the artificial implant 100 to expand radially.

The artificial implant 100 is connected to the inner shaft assembly, and their relative axial positions are restricted. After the artificial implant 100 is positioned in the body, the connection with the inner shaft assembly must be released so that the delivery system can detach from the artificial implant 100 and be withdrawn from the body. The inner shaft assembly can also control the timing and progress of expansion of the artificial implant 100. These operations can be achieved using the second handle 42, i.e., the second handle 42 is connected to the inner shaft assembly to control the release of the artificial implant 100, which release includes expansion, releasing the constraint of the inner shaft assembly on the artificial implant, and retrieval of the artificial implant.

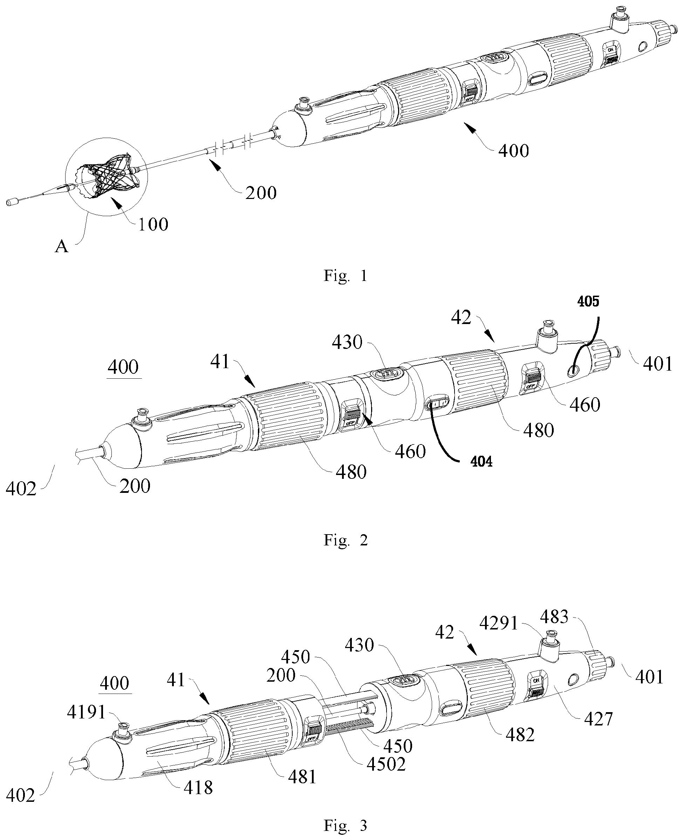

Referring to FIGS. 1 to 9, the present disclosure provides a delivery system for an artificial implant, having opposite distal and proximal ends. The delivery system comprises a control handle 400 and a catheter assembly 200 having a proximal end connected to the control handle 400. The artificial implant 100 is connected to a distal end of the catheter assembly 200 and is controlled by the control handle 400. The control handle 400 comprises a first handle 41 and a second handle 42 cooperating with each other. Each of the handles comprises a support member and a drive mechanism disposed on the support member. The drive mechanism comprises a plurality of sets, each coupled to a respective portion of the catheter assembly for controlling a wire control mechanism at the distal end of the catheter assembly to effect deployment, release, and retrieval of the artificial implant, or to effect steering of the catheter. The support members provide a base for the installation and movement of the drive mechanisms and/or the other handle, as well as structural support for the respective handles.

The support member of the first handle 41 comprises a first support body 410 and two support rods 450 fixedly connected to the first support body 410 and extending proximally. The support member of the second handle 42 comprises a second support body 420 slidably mounted on the support rods 450. Each support body is provided with a guide slot extending axially. The drive mechanism comprises a base movable axially along the control handle and a drive sleeve rotatably mounted on an outer periphery of the corresponding support body. The drive sleeve is threadedly engaged with the base. Some components of the catheter assembly are connected to the base and perform corresponding actions under the driving of the drive sleeve.

Each support body is provided, on a proximal side of its guide slot, with a rotational locking mechanism (which may be referred to as a second locking mechanism) configured to restrict or permit rotation of the drive sleeve. Of course, based on the same principle, the rotational locking mechanism may also be provided on the distal side of the guide slot.

A sliding locking mechanism, which may also be referred to as a first locking mechanism, is further provided on a distal side of the second support body 420 itself. The sliding locking mechanism interacts with the support rods 450 to maintain a relative position with respect to the first support body 410. The term "sliding" in "sliding locking mechanism" refers to the sliding movement of the second handle 42 relative to the first handle 41. The term "rotational" in "rotational locking mechanism" refers to the rotational movement of the drive sleeve relative to the corresponding support body.

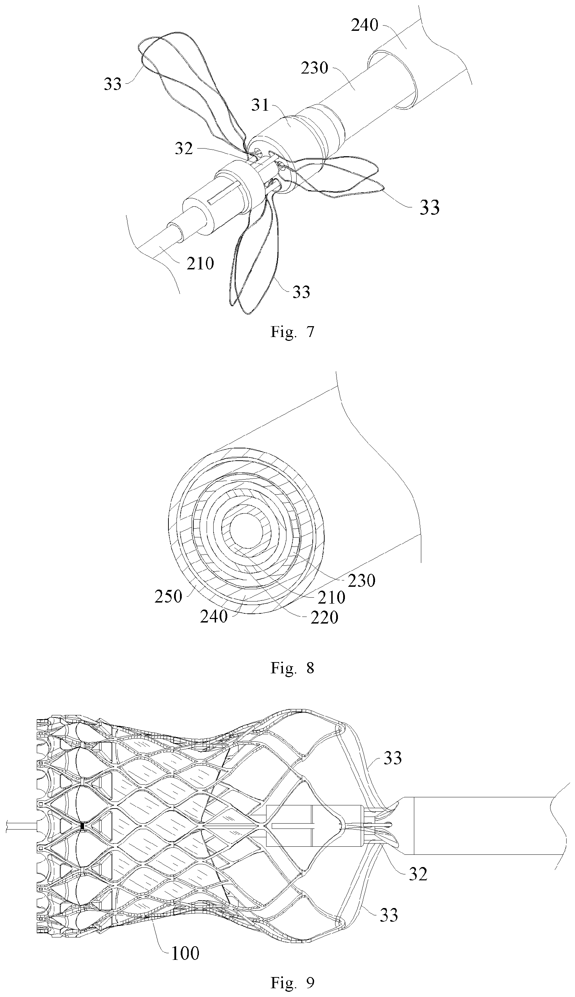

The catheter assembly 200 comprises, radially from outside to inside, an outer sheath 240 and an inner shaft assembly located within the outer sheath 240. The inner shaft assembly comprises at least one shaft, where "at least one shaft" means one shaft or a plurality of shafts fixed to each other. For example, two tubes fixed relative to each other may also be regarded as one shaft. As shown in FIG. 8, the catheter assembly 200 specifically comprises an outer sheath 240, a third shaft (e.g., the inner sheath tube 230 described below), a second shaft (e.g., the wire control tube 220 described below), and a first shaft (e.g., the inner core 210 described below). The first shaft 210, the second shaft 220, and the third shaft 230 belong to the inner shaft assembly. The distal end of the inner core 210 is provided with a tip.

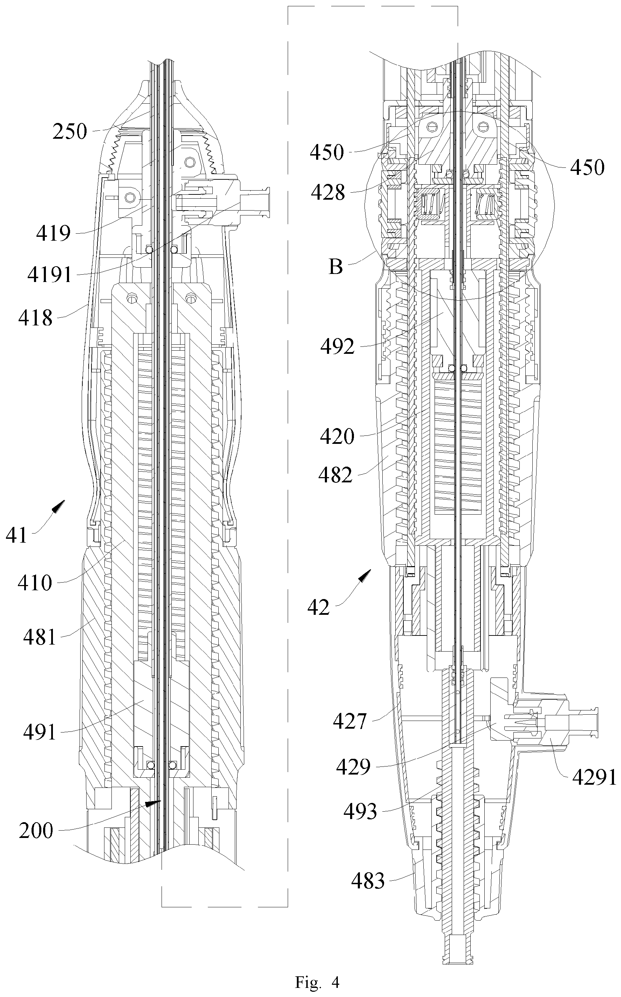

Referring to FIGS. 4 to 10, the proximal end of the outer sheath 240 is movably connected to a first base 491 of the first handle 41, and the distal end of the outer sheath 240 is configured to cover the artificial implant 100. The proximal end of the third shaft is fixed to the second handle 42. The proximal end of the second shaft is movably connected to a second base 492 of the second handle 42. The proximal end of the first shaft is movably connected to a proximal portion of the second handle 42. The inner shaft assembly, i.e., the first shaft, the second shaft, and the third shaft, is controlled by the second handle 42. When the sliding locking mechanism is unlocked, the second handle 42 can move away from or toward the first handle 41, thereby simultaneously driving rapid movement of the first shaft, the second shaft, and the third shaft relative to the outer sheath 240, so as to achieve closure between the proximal end of the tip and the distal end of the outer sheath.

In one embodiment, a first extension sleeve 418 is further fixed to the distal end of the first support body 410, and the catheter assembly 200 further comprises a jacket 250 disposed externally of the outer sheath 240, the jacket 250 having a proximal end fixed to the first handle 41.

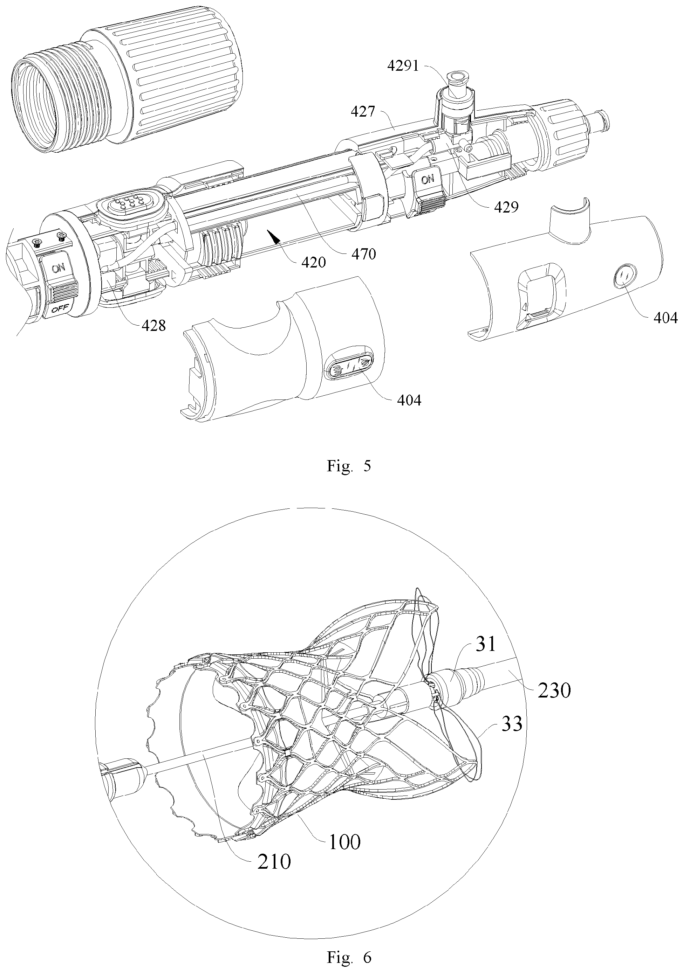

In one embodiment, the artificial implant is released and retrieved via a wire control mechanism. As shown in FIGS. 6, 7, 9, and 10, a lock base 31 is connected to a distal portion of the inner sheath tube 230. One end of the inner core 210 is an extension segment extending out of the distal end of the inner sheath tube 230, and a locking rod 32 is fixed to the extension segment, the locking rod 32 being located on a distal side of the lock base 31. A locking wire 33 is connected to a distal end of the wire control tube 220. The locking wire 33 is threaded through the artificial implant 100 and then tightened or loosened relative to the lock base 31 to constrain or release the artificial implant 100. The lock base 31, the locking wire 33, and the locking rod 32 together constitute the wire control mechanism.

When the artificial implant 100 is in a loaded state, the locking wire 33 extends out of the distal end of the inner sheath tube 230, passes around the artificial implant 100, and is then constrained to the locking rod 32. The locking rod 32 is inserted into the lock base 31 to prevent the locking wire 33 from being released. The proximal end of the locking wire 33 is connected to the control handle via the second shaft. The second shaft may be a wire control tube 220 or may be a wire; a plurality of wires may be provided, each corresponding to a locking wire 33. For example, the locking wire 33 and the second shaft may be an integral wire. In this embodiment and the accompanying drawings, the second shaft is exemplified as a wire control tube.

Relative movement between the inner core 210 and the inner sheath tube 230 can effect relative movement between the lock base 31 and the locking rod 32, thereby changing the constrained state of the locking wire 33. The state of the locking wire 33 can affect the movement of the artificial implant 100, particularly during the release process of the artificial implant 100. The locking wire 33 enables staged release of the artificial implant 100. Furthermore, the relative movement of the various tubes provides a structural basis for full release and full retrieval of the artificial implant 100, thereby providing a more controllable interventional procedure, improving treatment outcomes while enhancing patient experience. When the expanded artificial implant 100 (fully expanded but still connected to the locking wire 33) needs to be retrieved, unlocking the sliding locking mechanism allows the second handle 42 to slide relative to the first handle 41, enabling rapid retraction of the artificial implant 100 and its reception inside the outer sheath 240.

In one embodiment, the lock base is provided with one or more grooves along its circumference, and the extension end is provided with protruding structures that match the grooves in number and configuration. The locking wire is threaded through the artificial implant and is bundled by the grooves and protruding structures of the lock base. The wire control mechanism comprises a lock base, a locking wire, and a protruding structure cooperating with the lock base.

Referring to one embodiment, the lock base 31 is provided with a locking hole that cooperates with the locking rod 32. In the locked state, the locking rod 32 is inserted into the locking hole and restricts the range of motion of the locking wire 33. Referring to one embodiment, the locking rod 32 moves with the inner core 210 and has the following positions:

a locked position (see FIG. 9), in which the locking rod 32 is inserted into the locking hole to constrain the locking wire 33;

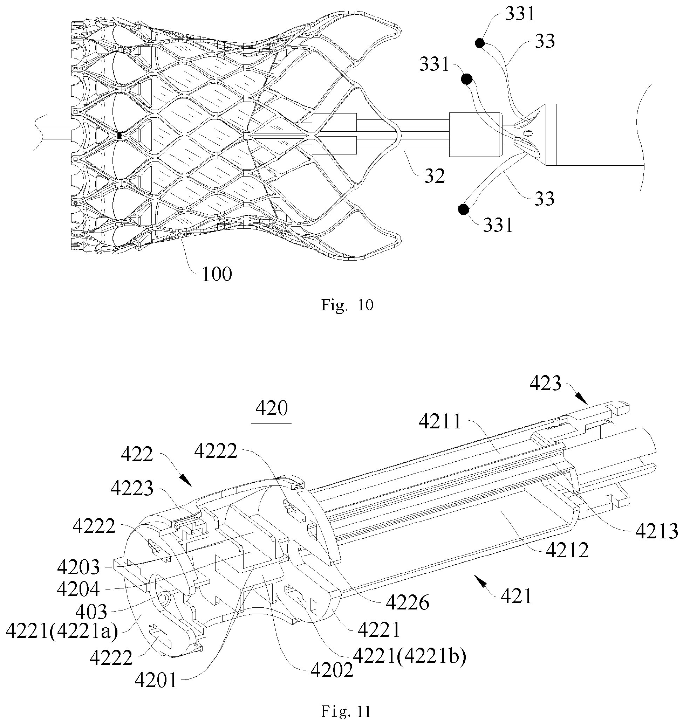

a released position (see FIG. 10), in which the locking rod 32 is withdrawn from the locking hole to release the locking wire 33.

In one embodiment, the catheter assembly 200 further comprises a wire control tube 220 slidably disposed externally of the inner core 210. One end of the locking wire 33 is a drive end connected to the wire control tube 220, and the other end of the locking wire is a working end. In the loaded state of the artificial implant, the working end passes around the artificial implant 100 and then cooperates with the locking rod 32. The locking wire 33 comprises a plurality of wires.

As shown in FIG. 10, in one embodiment, the locking wire 33 is provided with a radiopaque marker 331. The radiopaque marker 331 is disposed at the distal end of the locking wire 33 and is made of a radiopaque metal such as a platinum-iridium alloy or gold, facilitating intraoperative observation of the positional relationships among the locking wire 33, the artificial implant 100, the lock base 31, and the locking rod 32, thereby enabling precise release and retrieval operations. The radiopaque marker 331 is connected to the locking wire 33 by bonding, sewing, braiding, crimping, or other processes. The radiopaque marker 331 may have a solid or hollow structure and may have different shapes (e.g., annular, filamentary, circular, square, etc.) depending on the connection process.

During interventional procedures and in vitro simulation tests, for example, during interventional delivery, the rotational locking mechanism is in a locked state, and the outer sheath 240 covers the artificial implant 100 and the wire control mechanism until delivery to the treatment site, improving the safety of interventional delivery. When the artificial implant is being positioned and released, the rotational locking mechanism is unlocked, and the first handle 41 is operated to move the outer sheath 240 relative to the other catheter assembly components to control release of the artificial implant. After successful release of the artificial implant, the sliding locking mechanism is unlocked, and the second handle 42 is slid proximally relative to the first handle 41 to quickly retract the inner shaft assembly into the outer sheath, so that the tip just meets the distal end of the outer sheath, and then the assembly is withdrawn from the body, thereby improving operational efficiency and reducing safety risks during overall retraction.

As shown in FIG. 4, the control of the catheter assembly and the corresponding drive mechanisms is as follows:

The first support body 410 is provided with a first guide slot 4102. The second support body 420 is provided with a second guide slot 4212. A first extension sleeve 418 is fixed to the distal end of the first support body 410. A first support base 419 is fixed within the first extension sleeve 418. A first base 491 is slidably mounted in the first guide slot 4102 of the first support body 410, and a first drive sleeve 481 is rotatably sleeved on the outer periphery of the first support body 410 and threadedly engaged with the first base 491. A second extension sleeve 427 is fixed to the proximal end of the second support body 420. A second support base 428 is fixed to a distal portion of the second support body 420. A second base 492 and a third base 493 are slidably mounted in the second guide slot of the second support body 420. A second drive sleeve 482 and a third drive sleeve 483 are rotatably sleeved on the second support body 420 and are threadedly engaged with the second base 492 and the third base 493, respectively.

The jacket 250 is fixed to the first support base 419 within the first extension sleeve 418. The outer sheath 240 is fixedly connected to the first base 491. The inner sheath tube 230 is fixedly connected to the second support base 428. The wire control tube 220 sealingly extends through the second support base 428 and is fixedly connected to the second base 492. The inner core 210 is fixedly connected to the third base 493.

In one embodiment, the control handle 400 is provided with a venting structure (e.g., a one-way valve). Specifically, the first support base 419 is provided with a first venting structure 4191, and the second handle 42 is provided with a second venting structure 4291. In the illustrated embodiment, a third support base 429 is provided on the second extension sleeve 427, and the second venting structure 4291 is disposed on the third support base 429. The third support base 429 is connected to the second support base 428 by a vent tube 470, and the vent tube 470 extends through the second support body 420.

Specifically, the first venting structure is used to vent gas between the jacket and the outer sheath, and the second venting structure is used to vent gas between the outer sheath and the inner sheath tube, between the inner sheath tube and the wire control tube, and between the wire control tube and the inner core.

During a surgical procedure, the operator typically views the release or retrieval state of the artificial implant using imaging. However, the imaging system often provides poor visibility. To allow the operator to more intuitively understand the deployment or collapse state of the artificial implant at the surgical site, a viewing window indicating the deployment or collapse state of the artificial implant is provided in the control handle. For example, in one embodiment, as shown in FIG. 1, the control handle is provided with a first viewing window 404, which may be fitted with a transparent cover plate for observation and protection. The first viewing window 404 or its periphery displays patterns or text indicating the state of the artificial implant, for example: a fully deployed state of the artificial implant and a collapsed state of the artificial implant. Further, for example, the distal end of the first viewing window may bear an indicator for full stent expansion, and the proximal end may bear an indicator for stent collapse, to indicate the operational progress. The first viewing window 404 contains a movable indicator, such as a pointer, and the position of the indicator can be observed to determine the deployment or collapse state of the artificial implant. The indicator may be connected to the second base, and axial movement of the second base drives movement of the indicator.

As shown in FIG. 2, the control handle may further be provided with a second viewing window 405 for indicating whether the locking rod 32 has been disengaged from the locking wire, thereby indicating whether the prosthetic heart valve has been finally detached from the catheter assembly. The second viewing window 405 or its periphery displays patterns or text indicating whether the locking rod has been disengaged from the locking wire. Further, the second viewing window 405 contains a movable indicator, such as a pointer, and the position of the indicator can be observed to determine whether the locking rod has been disengaged from the locking wire. The indicator may be connected to the third base 492, and axial movement of the third base drives movement of the indicator.

As shown in FIGS. 4, 5, and 11, the second support body 420 comprises:
a second main body being cylindrical and extending axially, the second support body 420 being provided on radially opposite sides with axially extending third slide grooves 4211, each support rod 450 passing through a corresponding guide hole 4222 and being received in a corresponding third slide groove 4211;
a second connecting seat 422 comprising two partition plates 4221 and a transition portion 4223 connected between the two partition plates 4221, one of the partition plates 4221 being fixed to a distal end of the second main body 421;
a third connecting seat 423 fixed to a proximal end of the second main body 421.

The third slide grooves 4211 are provided on the back side of the second main body 421 to facilitate observation of the assembly of the support rods 450. Moreover, since the support rods 450 are strip-shaped, spatial occupation of the second main body 421 is reduced, ensuring the structural strength of the second main body 421. The second support base 428 is mounted inside the second connecting seat 422. The second main body 421 is provided with a groove 4213 serving as a passage. The partition plate at the proximal end (i.e., the second partition plate 4221b described below) is provided with a relief hole 4226. The vent tube 470, after communicating with the catheter assembly, extends from the second support base 428, passes through the relief hole 4226 and the groove 4213 after passing along the open side of the transition portion 4223, until it connects to the third support base 429. The groove 4213 is provided on an outer wall of the second guide slot 4212.

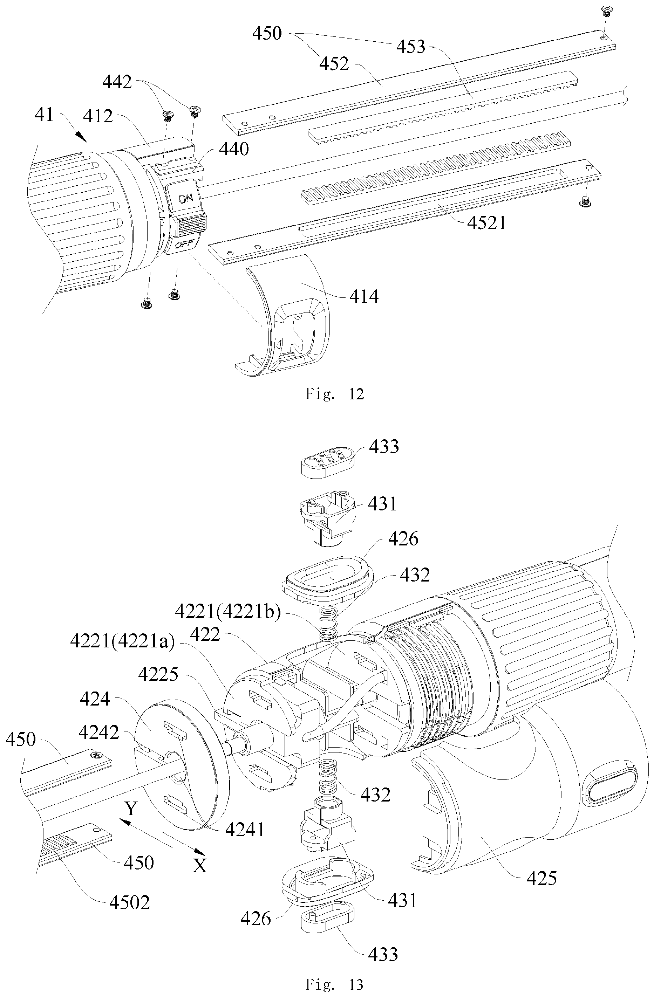

Referring to FIG. 13, in one embodiment, the two partition plates 4221 comprise a first partition plate 4221a at the distal end and a second partition plate 4221b at the proximal end. A cover plate 424 is engaged on a distal side of the first partition plate 4221a. The cover plate 424 is provided with a clearance region 4241 corresponding in position to the installation channel 403 and the guide holes 4222. The transition portion 4223 between the two partition plates 4221 is a housing structure having a semi-cylindrical surface. The second support body 420 further comprises a second half-shell 425 that engages with the transition portion 4223 to form a cylindrical shape. The cover plate 424 is engaged with the first partition plate 4221a and the second half-shell 425.

The distal side of the first partition plate 4221a is provided with a positioning rib 4225 extending radially. The cover plate 424 is provided with a positioning groove 4242 that cooperates with the positioning rib 4225. The two partition plates 4221 each extend radially open from their respective installation channel positions to their respective edges in a first radial direction X. The cover plate 424 extends radially open from its installation channel position to its edge in a second radial direction Y opposite the first radial direction X, thereby forming the positioning groove 4242.

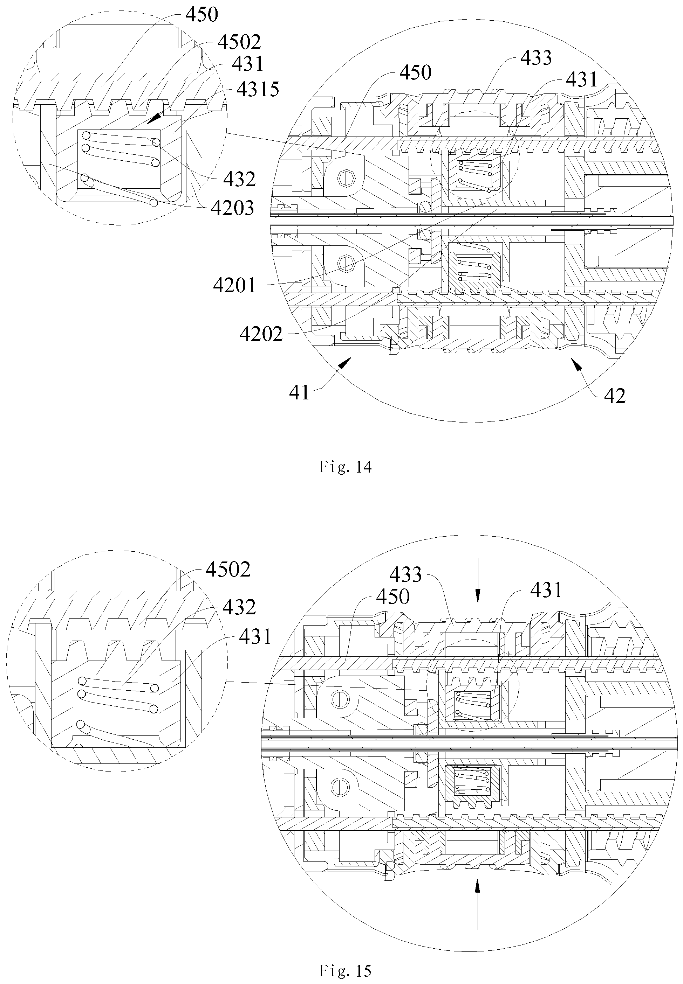

Referring to FIGS. 3 and 11 to 21, in one embodiment, two support rods 450 are arranged side by side, and a limiting member is arranged on the facing sides thereof. The sliding locking mechanism cooperates with the limiting member to maintain the relative position of the second handle with respect to the first support body. When the cooperation is released, the second handle is allowed to slide relative to the first support body. The limiting member is, for example, the positioning teeth 4502 provided in this embodiment. The support member of the second handle 42 comprises a second support body 420. The second support body 420 is provided with guide holes 4222, and the two support rods 450 extend into the respective guide holes 4222. The second handle 42 is further provided with a sliding locking mechanism 430 that cooperates with the positioning teeth 4502 to restrict the relative position of the first handle 41 and the second handle 42. As shown in FIG. 15, in the unlocked state, the sliding locking mechanism 430 allows the second handle 42 to slide along the support rods 450 relative to the first handle 41. As shown in FIG. 14, in the locked state, the position of the second handle 42 relative to the first handle 41 is fixed.

The support rod serves as a connecting member between the second handle and the first handle and is also provided with positioning teeth to cooperate with the sliding locking mechanism to control the position of the second handle relative to the first handle, satisfying the operational requirements of the control handle while simplifying the structure of the control handle.

The sliding locking mechanism 430 comprises a locking member 431, an elastic member 432, and an operating button 433. The locking member 431 is movably mounted on the second support body 420 and has a locked position engaged with the positioning teeth 4502 and an unlocked position disengaged from the positioning teeth 4502. The elastic member 432 (e.g., a spring) acts between the locking member 431 and the second support body 420, biasing the locking member 431 toward the locked position. The operating button 433 acts on the locking member 431, driving the locking member 431 toward the unlocked position. The specific unlocking and locking operations are as follows: a user applies force to the operating button 433, compressing the elastic member 432 and driving the locking member 431 to switch to the unlocked position; when the operating button 433 is released, the elastic member 432 returns and acts on the locking member 431, causing it to switch to the locked position.

In one embodiment, the locking member 431 and the operating button 433 slide radially. The locking member 431 is provided with a mounting hole 4311 formed by a slot in the interior of the locking member 431.

The locking member 431 is slidably sleeved on the support rod 450 through the mounting hole 4311. A clearance 4312 is provided between the mounting hole 4311 and the support rod 450 in a direction perpendicular to the sliding direction of the support rod 450 (i.e., the sliding direction of the locking member 431). The locking member 431 moves along the clearance 4312 when switching between the locked and unlocked positions. The clearance 4312 is configured such that the dimension of the mounting hole 4311 in the radial sliding direction of the locking member 431 is larger than the thickness of the support rod 450. Referring to FIGS. 13 to 17, during assembly, the locking member 431 is sleeved on the support rod 450, which prevents the locking member 431 from detaching from the second support body 420. By utilizing different installation directions and interactions among the components, additional limiting structures are omitted, simplifying the component structure and facilitating assembly.

In one embodiment, one side of the mounting hole 4311 faces the positioning teeth 4502, and that side is provided with an engaging portion 4313 that cooperates with the positioning teeth 4502. The engaging portion 4313 is a tooth structure that cooperates with the positioning teeth 4502. In conjunction with the direction of movement of the locking member 431, the locking member 431 moving radially outward tends toward the locked position, and moving radially inward tends toward the unlocked position. Thus, the engaging portion 4313 is provided on the bottom side of the mounting hole 4311 (which faces radially outward). The cross-sectional shape of the positioning teeth 4502 is trapezoidal.

In one embodiment, the number of positioning teeth 4502 and the operation of the control handle are as follows:
When the number of positioning teeth 4502 is one, it is used to maintain the second handle at the farthest position relative to the first handle to achieve closure between the proximal end of the tip and the distal end of the outer sheath (i.e., the second extreme position described below);
When the number of positioning teeth 4502 is plural and arranged at axial intervals, the second handle can be locked at multiple positions to adjust the position of the artificial implant within the body or to adjust the coverage distance of the outer sheath over the artificial implant during retrieval.

Two sliding locking mechanisms 430 are provided, each acting on a respective support rod 450, and the operating buttons 433 thereof are radially aligned with each other along a radial direction of the second handle 42 and are arranged in opposite directions. An abutment rib 4201 is fixed within the second support body 420, and the elastic member 432 is compressed and acts between the abutment rib 4201 and the locking member 431.

The control handle 400 has an installation channel 403 extending axially through the first support body 410. The abutment ribs 4201 of the two sliding locking mechanisms are spaced apart from each other to define a spacing region 4202. As shown in FIG. 11, the spacing region 4202 allows the installation channel 403 to extend therethrough.

In one embodiment, the locking member 431 is provided with a positioning seat 4315 on a side facing the elastic member 432, and the elastic member 432 is inserted into or sleeved onto the positioning seat 4315. In the illustrated embodiment, the elastic member 432 is a spring, and the positioning seat 4315 has a cavity for receiving a portion of the spring. After assembly, the elastic member 432 and the locking member 431 are prevented from detaching from the second support body 420 by the support rod 450. Two clamping plates 4203 protruding from the abutment rib 4201 and arranged at axial intervals define a mounting region 4204 that fits the positioning seat 4315, restricting axial movement of the locking member 431 and guiding the movement of the locking member 431.

In one embodiment, the support rod 450 is a metal strip having a strip-shaped cross-section in the radial direction of the control handle, which is substantially rectangular. The extension direction (length direction) of the cross-section is perpendicular to the spacing direction of the two support rods 450.

In one embodiment, the support rod 450 comprises a support strip 452 and a rack 453 having the positioning teeth 4502. As shown in FIG. 12, the support strip 452 is provided with a mounting groove 4521 for receiving the rack 453. The rack 453 may be connected to the support strip 452 by embedded fitting or fixed connection (e.g., adhesion or welding). The mounting groove 4521 is open toward the bottom side, and its extension range includes the sliding stroke of the second handle 42 relative to the first handle 41.

In one embodiment, the relationship between the positioning teeth 4502 and the bottom side of the support strip 452 is as follows:
the positioning teeth 4502 protrude beyond the bottom side of the support strip 452;
or the positioning teeth 4502 are flush with the bottom side of the support strip 452;
or the positioning teeth 4502 are below the bottom side of the support strip 452.

In one embodiment, the distal portion of the second support body 420 is a second connecting seat 422 comprising two partition plates 4221 and a transition portion 4223 connected between the two partition plates 4221.

The transition portion 4223 is a housing structure having a semi-cylindrical surface. The second support body 420 further comprises a second half-shell 425 that engages with the transition portion 4223 to form a cylindrical shape. The sliding locking mechanism 430 is positioned in a region enclosed by the transition portion 4223 and the second half-shell 425, with only a portion of the operating button 433 exposed for user operation.

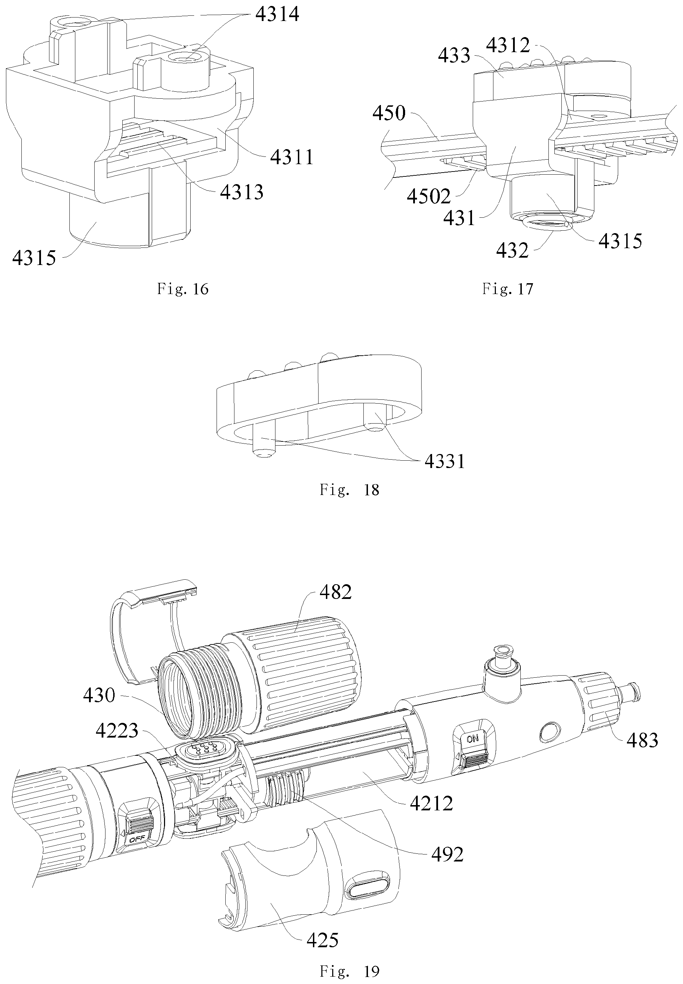

Referring to FIGS. 16 and 18, in one embodiment, the operating button 433 and the locking member 431 are engaged and fixed together by a snap-fit connection. One of them is provided with a snap post, and the other is provided with a snap hole that cooperates with the snap post. For example, in the illustrated embodiment, snap posts 4331 are protrudingly provided on the operating button 433, and snap holes 4314 are provided in the locking member 431 and are in tight fit with the snap posts 4331. Two snap posts 4331 are provided.

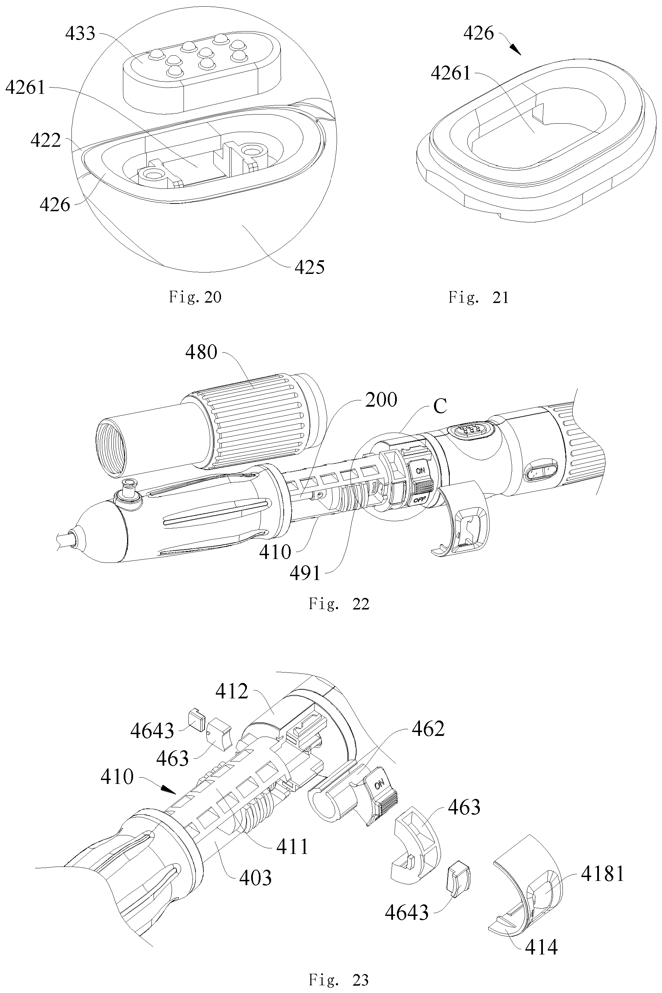

Referring to FIGS. 20 and 21, in one embodiment, the second support body 420 further comprises a snap-fit seat 426 engaged between the second connecting seat 422 and the second half-shell 425. The snap-fit seat 426 has an open slot 4261 through which the operating button 433 is exposed. The snap-fit seat 426 surrounds the operating button 433 circumferentially and fills the gap between the operating button 433 and the second support body 420 and the second half-shell 425.

The open slot 4261 is circumferentially closed, and the slot wall extends along the direction of movement of the operating button 433, providing guidance for the movement of the operating button 433.

Referring to FIGS. 2 and 23 to 38, the rotational locking mechanism 460 comprises a first tubular member 461, a second tubular member 462, and a locking member 463. It should be noted that the first tubular member 461 and the second tubular member 462 are not part of the catheter assembly 200. The first tubular member 461 is fixed to the support body, for example, by being separately connected and fixed inside the handle, or by being integrally formed as part of the support body. The illustrated embodiment takes the first support body 410 as an example, and the first tubular member 461 is part of the first support body 410. A portion of the installation channel 403 extends through the interior of the first tubular member 461, i.e., the first tubular member 461 is hollow and allows part or all of the catheter assembly 200 to pass therethrough.

The second tubular member 462 is rotatably sleeved on the outer periphery of the first tubular member 461. A portion of the second tubular member 462 is a working section 4621 extending into the rotating member 480, and the working section 4621 has a protrusion 4622 on its outer wall. Both the first tubular member 461 and the second tubular member 462 extend axially of the control handle. They are not strictly required to be circumferentially closed and may be locally open; their cross-sectional shapes are not strictly limited either.

The locking member 463 is positioned within a radial gap between the working section 4621 and the rotating member 480 and is configured to be movable radially relative to the rotating member 480 and to act against an inner wall of the rotating member 480. During rotation of the second tubular member 462, the protrusion 4622 presses against the locking member 463, causing it to move and thereby lock the rotating member 480, or the protrusion 4622 releases the pressure on the locking member 463 to unlock the rotating member 480. The protruding direction of the protrusion 4622 includes at least the radial direction.

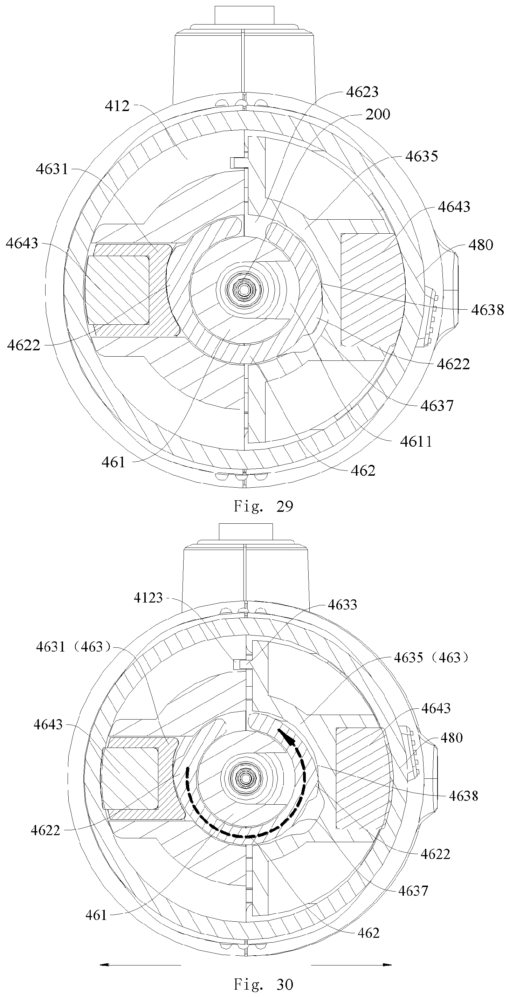

As shown in FIG. 29, in the unlocked position, there is a clearance between the locking member 463 and the inner wall of the rotating member 480, allowing the rotating member 480 to be easily rotated manually. As shown in FIG. 30, when the second tubular member 462 is rotated about the dashed line to the locked position, the locking member 463 moves in the direction of the arrow to abut against the rotating member 480, generating a sufficiently large force (e.g., static friction) therebetween, thereby restricting rotation of the rotating member 480. This structure can lock the rotating member 480 at any position, which is advantageous for controlling the artificial implant. Compared to existing locking structures, it improves the operational precision of the control handle, and the locking operation is accomplished in one step, simplifying the operation steps. Furthermore, the locking member is installed in the radial gap between the working section and the rotating member, providing a clean appearance of the control handle, facilitating grasping and operation, effectively utilizing space, and making the control handle more compact.

In this embodiment, the rotational locking mechanism 460 further comprises an operating member 464 for driving rotation of the second tubular member 462. The operating member 464 is movably disposed on the first support body 410 and may be connected to the second tubular member 462 as follows:
a) separately connected components;
b) integrally formed, e.g., the second tubular member 462 and the operating member 464 are integrally formed, with one axial segment of the second tubular member 462 being the working section 4621 and the other segment being connected to the operating member 464;
c) independent of each other, with the two components being operatively coupled via a transmission member.

The second tubular member 462 has opposite positions:
a locked position, in which the protrusion 4622 is pressed against the locking member 463, thereby acting on the rotating member 480 and generating a pressing force thereagainst;
a released position, in which the protrusion 4622 releases the pressing force against the locking member 463.

As shown in FIGS. 22 to 24, in one embodiment, a side wall of the first support body 410 and a side wall of the first tubular member 461 are locally open on the same radial side to form an installation opening 4611. The installation opening 4611 communicates with the installation channel 403, facilitating installation of the catheter assembly 200 and the base.

In one embodiment, a side wall of the second tubular member 462 is open on one radial side, and the open position is circumferentially offset from the open position of the first tubular member 461. The open side of the second tubular member 462 allows its working section 4621 to have a certain degree of radial deformability. For example, in the locked state, the inner wall of the working section 4621 abuts against the outer wall of the first tubular member 461, and the working section 4621 itself is subjected to forces from both the radially inner and outer sides, thereby restricting rotation of the second tubular member 462. In the unlocked state, a relatively large driving force must be applied to the operating member to release the lock, thereby preventing accidental unlocking and improving safety.

The fact that the open position of the second tubular member 462 is circumferentially offset from the open position of the first tubular member 461 means that, taking the unlocked position of FIG. 29 as an example, the opening 4623 of the second tubular member 462 does not coincide with the installation opening 4611 during rotation of the second tubular member 462.

Referring again to FIGS. 23 to 27, in one embodiment, the first support body 410 comprises a first main body 411 and a first connecting seat 412. The first main body 411 is cylindrical and extends axially of the control handle. The first tubular member 461 is located on a proximal side of the first main body 411. The first connecting seat 412 is fixed to the proximal end of the first main body 411, leaving a radial gap between the first connecting seat 412 and the first tubular member 461. The second tubular member 462 is radially positioned within the radial gap. The first connecting seat 412 is a housing structure having a semi-cylindrical surface. The first support body 410 further comprises a first half-shell 414 that engages with the first connecting seat 412 to form a cylindrical shape. The first half-shell 414 is provided with a clearance window 4181, through which the operating member 464 is exposed. The operating member 464 is provided with an anti-slip portion 4641 and indicators 4642 indicating locked and unlocked states.

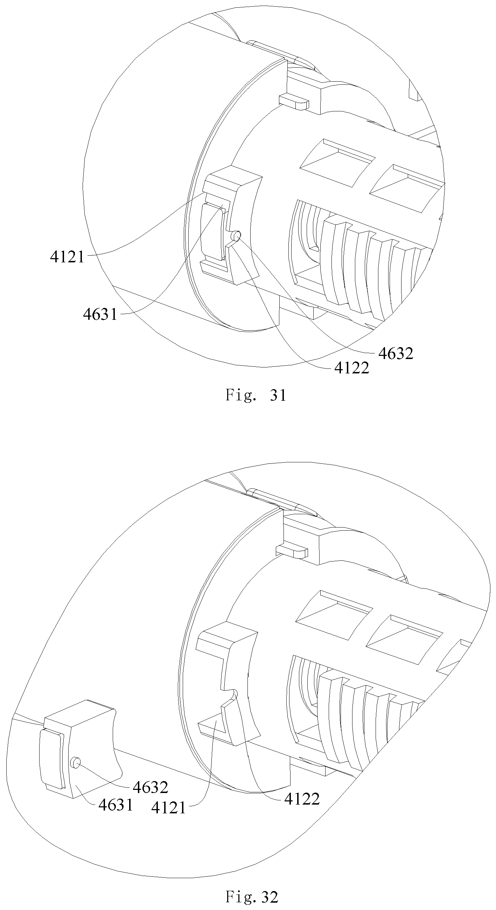

The arrangement of the locking member is as follows:
As shown in FIGS. 31 and 32, in one embodiment, the first connecting seat 412 is provided with a guide slot 4121 extending radially therethrough. The locking member comprises a first locking member 4631 (e.g., a block) slidably disposed in the guide slot 4121 in the radial direction. The protrusion 4622 on the second tubular member 462 abuts against a radially inner side of the first locking member 4631. The outer contour of the working section 4621 of the second tubular member 462 is circular. The protrusion 4622 extends along the rotational direction of locking (hereinafter referred to as the first direction), and the height of the outer surface of the protrusion 4622 gradually increases along the first direction. The inner wall of the locking member is an arcuate surface that cooperates with the outer contour of the protrusion 4622.

Based on the cooperating shape and structure of the locking member 463 and the protrusion 4622 described above, a registration structure is provided on a groove wall of the guide slot 4121 to cooperate with the first locking member 4631. The registration structure prevents incorrect installation of the first locking member 4631 and positions the first locking member 4631 relative to the guide slot 4121. The registration structure comprises:
a positioning groove 4122 provided on either the groove wall of the guide slot 4121 or the first locking member 4631; and
a positioning block 4632 provided on the other of the groove wall of the guide slot 4121 or the first locking member 4631 and cooperating with the positioning groove 4122. The positioning groove 4122 has a flared configuration.

As shown in FIGS. 30, 33, and 34, in another embodiment, the locking member 463 may comprise a plurality of components circumferentially arranged, for example, including the first locking member 4631 and a second locking member 4635. The second locking member 4635 is radially movably engaged with the first connecting seat 412. The first locking member 4631 and the second locking member 4635 move synchronously in opposite radial directions, acting on different radially inner walls of the same rotating member 480, thereby increasing locking strength.

The second locking member 4635 is semi-annular, and a cooperating guide structure is provided between the second locking member 4635 and the first support body 410.

As shown in FIG. 30, the guide structure comprises:
a first sliding portion 4633 provided on either the first connecting seat 412 or the second locking member 4635; and
a first slide groove 4123 provided on the other of the first connecting seat 412 or the second locking member 4635 and cooperating with the first sliding portion 4633.

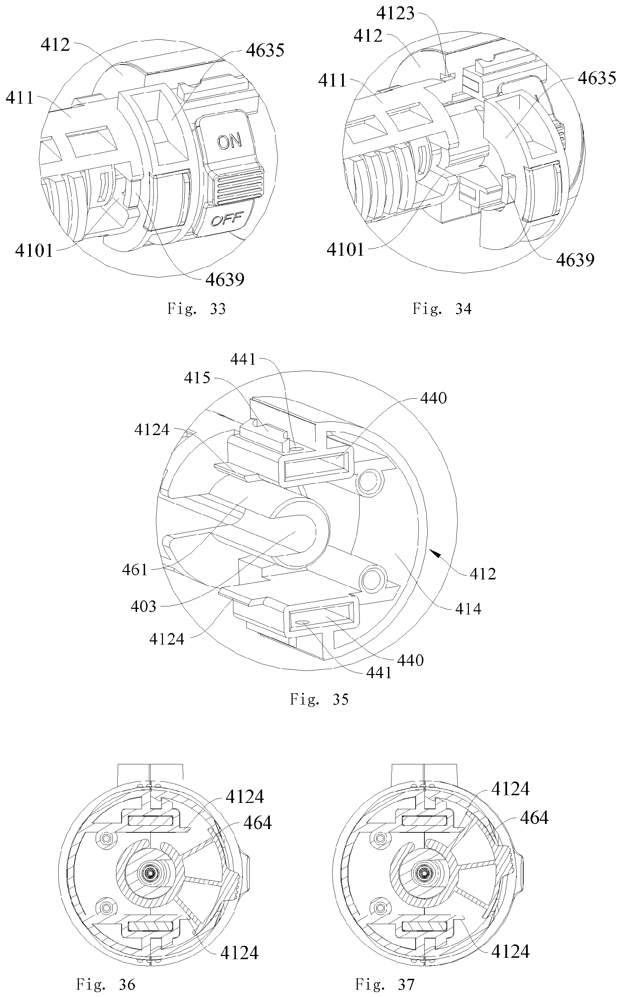

As shown in FIGS. 33 and 34, in another embodiment, the guide structure further comprises:
a second slide groove 4101 provided on the first main body 411; and
a second sliding portion 4639 provided on the second locking member 4635 and cooperating with the second slide groove 4101.

Referring again to FIGS. 29 and 30, in one embodiment, the first locking member 4631 and the second locking member 4635 are at the same axial position and are located on radially opposite sides of the second tubular member 462. In another embodiment, a plurality of locking members 463 are arranged along the axial direction, e.g., multiple locking members acting on different axial positions of the same rotating member.

A stop member 4643 is embedded at a position where the locking member 463 cooperates with the rotating member 480. With reference to the above, both the first locking member 4631 and the second locking member 4635 are embedded with a stop member 4643, as shown in FIG. 23. The first locking member 4631 and the second locking member 4635 are made of a hard material, allowing them to slide stably after being fitted with the first connecting seat 412. The stop member 4643 is made of a soft material having a high coefficient of friction and a certain degree of deformability, such as rubber. In the locked state, the stop member 4643 is pressed against the inner wall of the rotating member 480, thereby increasing static friction.

Regarding the second locking member 4635, it is installed in the gap between the first connecting seat 412 and the first half-shell 414, and can move radially via the guide structure described above, at least ensuring that there is always a gap between the second locking member 4635 and the inner wall of the rotating member 480. Thus, in the unlocked position, the rotating member 480 can rotate smoothly.

The inner wall of the second locking member 4635 has a radially undulating structure comprising an arcuate surface 4638 (centered on the axis of the control handle) and, in the unlocked position, a relief region 4637 that is radially recessed to accommodate a portion of the protrusion 4622. As shown in FIG. 29, in the unlocked position, the segment of the protrusion 4622 having a greater radial height is positioned within the relief region 4637. As shown in FIG. 30, in the locked position, that segment abuts against the arcuate surface 4638 and drives the second locking member 4635 to slide radially outward.

As shown in FIGS. 35 to 37, in one embodiment, the first connecting seat 412 has a stop portion 4124 that abuts against the operating member 464 to limit its rotational range. In the illustrated embodiment, there are two stop portions 4124, each being a plate-like structure. The operating member 464 abuts against one of the stop portions 4124 in the locked position and against the other stop portion 4124 in the unlocked position.

In one embodiment, a portion of the first support body 410 is radially open to form a first guide slot 4102. A base (e.g., the first base 491) is slidably disposed in the first guide slot 4102, the base being configured to be connected to a controlled member (e.g., the catheter assembly 200). The rotating member 480 is a drive sleeve rotatably sleeved on an outer periphery of the first support body 410, with the inner wall of the drive sleeve being threadedly engaged with the base. Thus, the stop member 4643 can better adapt to the threaded structure of the drive sleeve.

Regarding the rotational locking mechanism, it is independent of the number of handles. For example, the control handle 400 may comprise a single handle provided with a rotational locking mechanism.

In one embodiment, two connecting sleeves 440 are fixedly connected to a proximal portion of the first support body 410. A distal portion of each support rod 450 is fixedly inserted into a corresponding connecting sleeve 440. The fixation may be achieved by a snap-fit or by fasteners.

The support member of the second handle 42 comprises a second support body 420. The distal portion of the second support body 420 includes two partition plates 4221 arranged at axial intervals. Each partition plate 4221 is provided with a guide hole 4222. The second support body 420 is provided on radially opposite sides with axially extending third slide grooves 4211. Each support rod 450 passes through a corresponding guide hole 4222 and is received in a corresponding third slide groove 4211. The second handle 42 as a whole is slidably fitted along the two support rods 450 relative to the first handle 41 and has two extreme positions where it moves toward or away from the first handle 41: a first extreme position where the second handle 42 abuts against the first handle 41 (as shown in FIG. 2), and a second extreme position where the second handle 42 is spaced apart from the first handle 41 (as shown in FIG. 3).

The support rod is rod-shaped, which facilitates manufacturing and reduces processing costs. Moreover, a wider range of materials is available; for example, hard metals can be used to increase the structural strength of the support member of the first handle and to improve the connection stability between the first handle and the second handle, i.e., the support rods are less likely to bend or break when the second handle is moved away from the first handle.

In addition, to ensure sliding stability of the second handle, two support rods are provided. Moreover, due to the structural shape of the support rods, their cross-sectional area is small, occupying less space and facilitating arrangement within the interior space of the second handle.

The control handle 400 has an installation channel 403 extending axially through the first support body 410 and the second support body 420. The installation channel 403 allows the catheter assembly 200 to pass therethrough. The first support body 410 comprises a first main body 411 and a first connecting seat 412. The first main body 411 is cylindrical and extends axially. The first connecting seat 412 is fixed to a proximal end of the first main body 411. Two connecting sleeves 440 are fixed to the first connecting seat 412 and are located on radially opposite sides of the installation channel 403. Specifically, the connecting sleeve 440 is integrally formed with the first main body 411 and is open at its proximal end for insertion of the support rod 450.

The connection between the support rod 450 and the connecting sleeve 440 is as follows:
As shown in FIGS. 12, 35, and 38, a side wall (radially outer side) of the connecting sleeve 440 and the distal end of the support rod 450 are provided with positioning holes 441 at corresponding positions, and are fixedly connected by a connector 442 extending through the positioning holes 441. For example, the connector 442 may be a screw, and the support rod 450 may be provided with a threaded hole that cooperates with the screw.

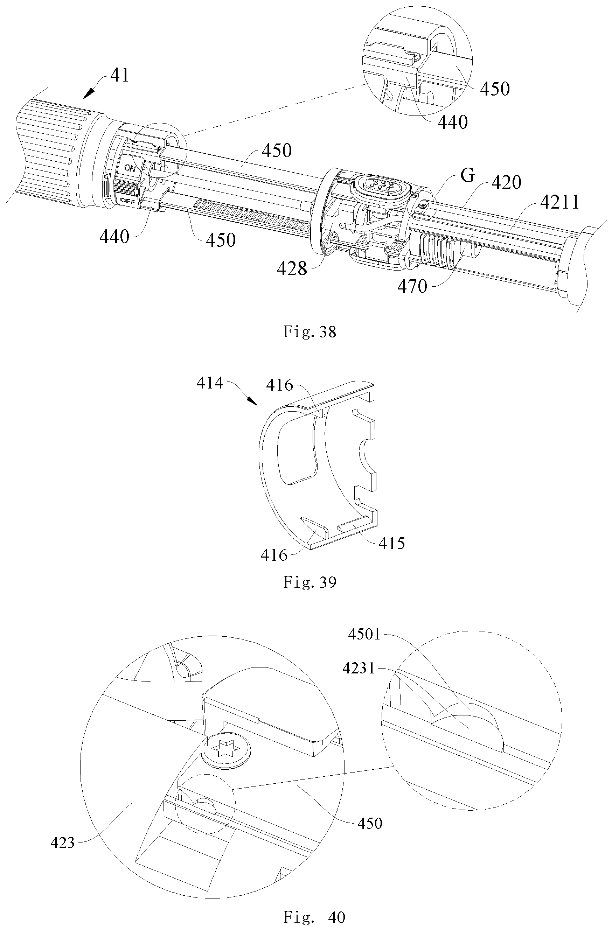

As shown in FIGS. 35 and 39, in one embodiment, the first connecting seat 412 is a housing structure having a semi-cylindrical surface. The housing structure serves as a portion of the outer shell and can be grasped. The first support body 410 further comprises a first half-shell 414 that engages with the first connecting seat 412 and encloses the housing structure to form a cylindrical shape. The connecting sleeve 440 is positioned at the interface between the first connecting seat 412 and the first half-shell 414, and an outer wall of the connecting sleeve 440 is provided with a snap-fit feature 415 that cooperates with the first half-shell 414.

In a preferred embodiment, the first half-shell 414 is installed in a radial direction, and an inner wall of the first half-shell 414 is provided with a limiting rib 416 that abuts against the connecting sleeve 440 to restrict axial movement of the first half-shell 414. Specifically, after the first half-shell 414 is assembled, the limiting rib 416 abuts against an end face of the snap-fit feature 415 on the connecting sleeve 440 in the axial direction of the control handle.

The first support body 410 partially serves as the outer shell, reducing manufacturing costs and simplifying the connection to the outer shell.

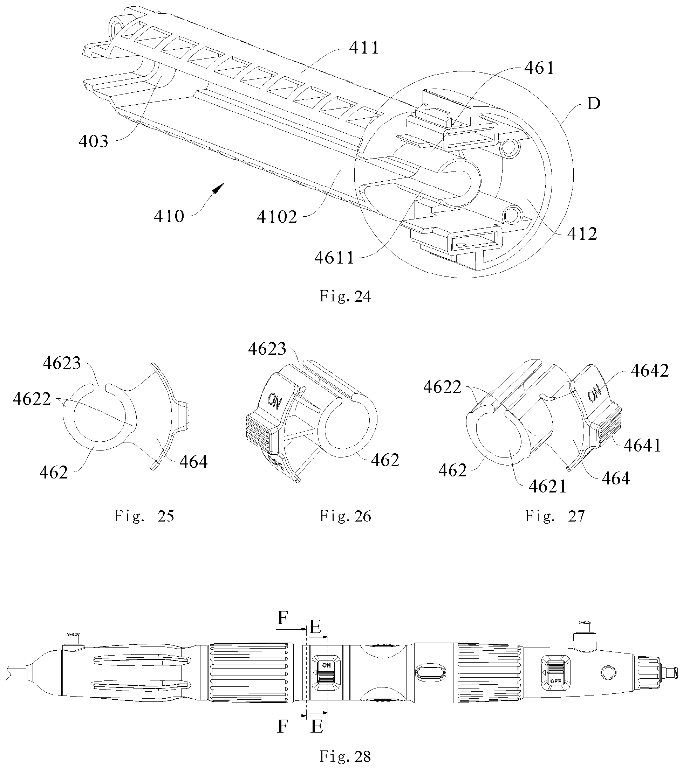

Referring again to FIGS. 24 and 35, in one embodiment, the first connecting seat 412 is disposed on a side opposite an opening of the first guide slot 4102, and a line connecting the two connecting sleeves 440 is arranged at 90 degrees to a direction of the groove opening.

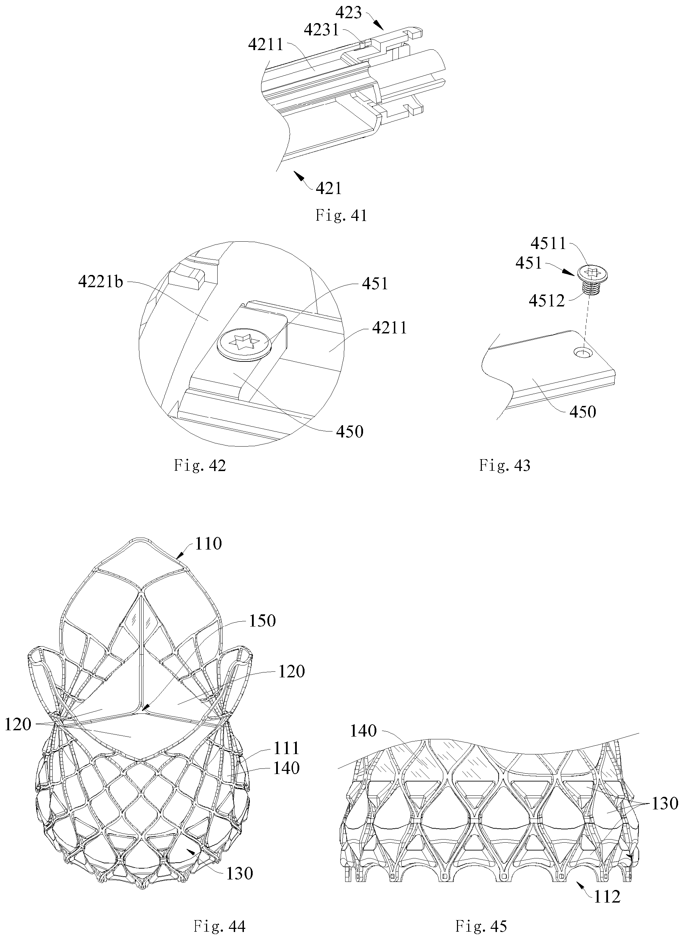

As shown in FIGS. 40 and 41, in one embodiment, a proximal portion of the third slide groove 4211 on the second main body 421 is a terminal section extending to an outer periphery of the third connecting seat 423. A cooperating snap-fit structure is provided between a groove wall of the terminal section and the support rod 450. Specifically, the snap-fit structure comprises:
an engagement block 4231 protruding from the groove wall of the third slide groove 4211;
a snap-fit groove 4501 provided on the support rod 450 and cooperating with the engagement block 4231. When the second handle 42 is in the first extreme position, the engagement block 4231 and the snap-fit groove 4501 engage with each other. That is, the snap-fit groove 4501 is provided near a proximal end of the support rod 450. This snap-fit structure provides only a certain amount of resistance. After the sliding locking mechanism is unlocked, applying a certain external force to the second handle can disengage the snap-fit structure. This snap-fit structure prevents the second handle from sliding when an operator accidentally unlocks the sliding locking mechanism, thereby improving safety.

As shown in FIGS. 42 and 43, in one embodiment, an anti-detachment member 451 is provided at a proximal portion of the support rod 450. When the second handle slides proximally relative to the first handle to the extreme position (i.e., the second extreme position), the anti-detachment member 451 is stopped by the partition plate. The anti-detachment member 451 may be integrally formed with the support rod 450 or separately fixed. For example, the anti-detachment member 451 may be a screw, where the screw includes a screw rod 4512 screwed into the support rod 450 and a nut 4511 protruding above an upper surface of the support rod 450. When the second handle 42 is in the second extreme position, the nut 4511 abuts against an end surface of the second partition plate 4221b. During assembly, the support rod 450 is first inserted through the second partition plate 4221b, and then the anti-detachment member 451 is installed.

Referring to FIGS. 44 and 45, the present disclosure also provides an artificial implant 100 having a tubular stent 110 with a mesh structure 111. The interior of the stent 110 defines a blood flow passage 150. A plurality of leaflets 120 within the stent cooperate with each other within the blood flow passage to relatively open or close the blood flow passage 150. The stent 110 has an axial direction (axis direction), a radial direction perpendicular to the axial direction, and a circumferential direction arranged around the axis. The mesh structure can be understood as the tubular side wall having hollowed-out regions, the edges of which form the struts defining the mesh. The size and specific shape of the mesh are not strictly limited. In the axial direction of the stent, the inflow side 112 and the opposite outflow side correspond to the normal direction of blood flow in the body. The stent itself may be formed by tube cutting or braiding, and the material is selected according to the manner of deployment in the body (e.g., self-expanding or balloon-expandable).

The artificial implant 100 further comprises an inner skirt 140 connected to the stent 110 and positioned within the blood flow passage 150. The inner skirt 140 is generally attached to the corresponding struts of the stent by suturing. The outflow side of the inner skirt 140 is joined to the inflow side of each leaflet 120, for example by suturing. The artificial implant 100 further comprises a plurality of sealing members 130 arranged circumferentially around the stent 110 and embedded in corresponding mesh structures 111. The plurality of sealing members 130 are circumferentially closed around the stent 110, and all mesh structures in the same row are filled and embedded by the sealing members 130. The sealing members protrude outwardly from the corresponding mesh structures to interact with surrounding tissue in the body to prevent blood from passing around the outer circumference of the artificial implant. The sealing members 130 are connected to the inner skirt 140 by at least one of bonding, thermal fusion, suturing, impregnation, or dip coating. The lock base 31, locking wire 33, and locking rod 32 together constitute the wire control mechanism.

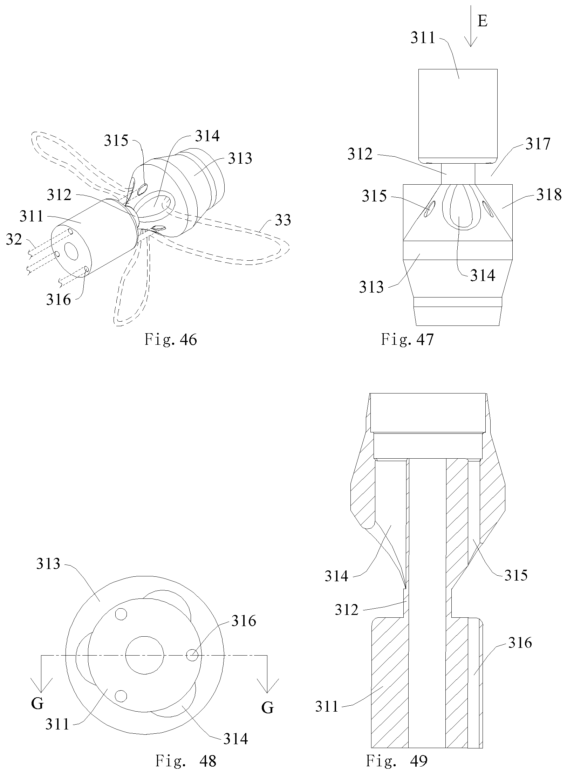

The present disclosure also provides a novel wire control mechanism for locking and releasing a prosthetic heart valve. As shown in FIGS. 46, 47, 48, and 49, the wire control mechanism for an artificial implant has opposite distal and proximal ends and comprises:
a lock base 31 comprising, from the distal end to the proximal end, a guide portion 311, a reduced-diameter portion 312, and a connecting portion 313. The connecting portion 313 is provided with a locking hole 315 and a wire hole 314, respectively. The guide portion 311 is provided with a guide hole 316 corresponding in position to the locking hole 315. The reduced-diameter portion 312 has an outer periphery defining a radially open engagement region 317. The distal end of the connecting portion 313 converges in shape and forms, on its outer periphery, an expansion region 318 communicating with the engagement region 317;
a locking rod 32 slidably fitted with the lock base 31 and inserted into the locking hole 315 via the guide hole 316;
a locking wire 33 having a drive end at one end extending proximally through the wire hole 314, and a working end at the opposite end configured to be threaded through the artificial implant and then coupled to the locking rod 32.

The structure of the lock base 31 in the prior art is shown in FIG. 7: the lock base 31 includes a guide portion 311, a reduced-diameter portion 312, and a connecting portion 313. The connecting portion 313 is substantially cylindrical and is provided with a locking hole 315 and a wire hole 314. When the locking rod 32 is inserted into the locking hole 315 of the connecting portion 313 via the guide hole 316 of the guide portion 311, precise alignment is required. Because the dimensions of the locking rod 32, the locking hole 315, and the guide hole 316 are all small, the processing precision requirements are extremely high. Moreover, when the working end of the locking wire 33 is threaded around the artificial implant, the operation is performed in the peripheral region of the reduced-diameter portion 312 (i.e., the engagement region 317), where the working space is small and the line of sight is easily obstructed.

In the present disclosure, the structure of the lock base 31 is shown in FIGS. 46 to 48. The distal end of the connecting portion 313 of the lock base 31 converges in shape and forms, on its outer periphery, an expansion region 318 communicating with the engagement region 317. The distal ends of the locking hole 315 and the wire hole 314 of the connecting portion 313 are exposed within the expansion region 318. The extent of the expansion region 318 is indicated by the dashed-line box in FIG. 47, which is a region surrounding the connecting portion 313 with a triangular cross-section. This region provides additional space for operating the locking wire 33 compared to the engagement region 317 around the lock base 31 in the prior art. As shown in FIG. 49, both the locking hole 315 and the wire hole 314 of the connecting portion 313 are exposed within the expansion region 318. The locking wire 33 exits through the wire hole 314 and is connected to the artificial implant. The opening of the wire hole 314 is relatively large, making entanglement between multiple locking wires 33 less likely, and providing better visibility when threading through the artificial implant.

As shown in FIG. 49, the opening of the locking hole 315 is also relatively large, and the opening of the locking hole 315 lies on an inclined surface. When the locking rod 32 is to be inserted into the locking hole 315, it is guided by the inclined surface before entering the locking hole 315, reducing the difficulty of alignment.

The present disclosure also provides a delivery system for an artificial implant, having opposite distal and proximal ends. The delivery system comprises a control handle 400 and a wire control mechanism having a proximal end connected to the control handle 400. The artificial implant is connected to a distal end of the wire control mechanism and is controlled by the control handle 400.

The control handle 400 comprises a support body (i.e., the first support body 410) provided with a guide slot (i.e., the first guide slot 4102), a base (i.e., the first base 491) movable along the guide slot, and a drive sleeve (i.e., the first drive sleeve 481) rotatably mounted on an outer periphery of the support body, the drive sleeve being threadedly engaged with the base.

The support body is provided, on a proximal side of its guide slot, with a rotational locking mechanism 460 configured to restrict or permit rotation of the drive sleeve. The rotational locking mechanism 460 comprises:
a first tubular member 461 fixed to the support body, wherein the control handle 400 has an installation channel 403 extending axially through the support body, a portion of the installation channel extending through the interior of the first tubular member 461;
a second tubular member 462 rotatably sleeved on an outer periphery of the first tubular member 461, a portion of the second tubular member being a working section 4621 extending into the drive sleeve, the working section 4621 having a protrusion 4622 on an outer wall thereof;
a locking member 463 positioned within a radial gap between the working section 4621 and the drive sleeve, and configured to be slidable radially relative to the drive sleeve and to act against an inner wall of the drive sleeve, wherein upon rotation of the second tubular member 462, the protrusion 4622 presses against the locking member 463 to move the locking member and thereby lock the drive sleeve;
an operating member 464 configured to drive rotation of the second tubular member 462.

The delivery system for an artificial implant further comprises:
an inner sheath tube 230, the lock base 31 being fixed to a distal end of the inner sheath tube 230;
an inner core 210 slidably extending through the inner sheath tube 230, the distal end of the inner core 210 passing out of the lock base 31, and a locking rod 32 being fixed to the portion that passes out, the locking rod 32 moving with the inner core 210 and having the following positions:
a locked position, in which the locking rod 32 is inserted into the locking hole 315 to constrain the working end of the locking wire 33;
a released position, in which the locking rod 32 is withdrawn from the locking hole 315 to release the working end of the locking wire 33.
a wire control tube 220 slidably extending through the radial gap between the inner core 210 and the inner sheath tube 230, the drive end of the locking wire 33 being connected to the wire control tube 220.

There are a plurality of locking rods 32, each having a different length. In the released position, the proximal ends of all locking rods 32 are located within the guide hole 316 of the guide portion 311. As shown in FIG. 47, there are at least two locking rods 32, or three, or more. The lengths of the locking rods 32 are different from each other. The axial length of the guide portion 311 is made sufficiently long so that when the locking rods 32 are in the released position, the proximal ends of all locking rods 32 are also located within the guide hole 316 of the guide portion 311, rather than being exposed outside the guide portion 311. Since the proximal ends of the locking rods 32 remain continuously within the guide hole 316, the locking rods 32 can maintain a connection with the lock base 31 at all times, allowing the locking rods 32 to switch between the locked position and the released position more smoothly and safely.

The present disclosure also provides a delivery system for an artificial implant having a steering function. The delivery system has opposite distal and proximal ends. The delivery system comprises a control handle 400 and a catheter assembly 200 having a proximal end connected to the control handle 400. The artificial implant 100 is connected to a distal end of the catheter assembly 200 and is controlled by the control handle 400. The control handle 400 comprises a first handle 41 and a second handle 42 cooperating with each other. Each of the handles comprises a support member and a drive mechanism disposed on the support member. The first handle is configured to drive relative movement of the outer sheath 240 to effect deployment or collapse of the artificial implant, for example, by moving the outer sheath proximally to deploy the artificial implant folded or compressed within the outer sheath. Unlike the previous embodiments, this embodiment further includes a steering member, which may be a steering tube or a steering wire. The second handle 42 is provided with a steering drive mechanism configured to drive movement of the steering member, thereby steering the distal end of the catheter assembly by tensioning the steering member. Specifically, the steering drive mechanism may be implemented in the manner described in the above embodiments. The distal end of the steering member may be connected to the outer sheath or the inner shaft assembly, and the proximal end of the steering member is fixed to the second base 492. By the threaded engagement of the second drive sleeve 482 with the second base 492, movement of the steering member is achieved, thereby changing the distal orientation of the outer sheath or the inner shaft assembly.

In one embodiment, the second handle 42 is further provided with a release drive mechanism for controlling release of the artificial implant. For example, the release drive mechanism is a control mechanism that drives a control wire 34. The control wire 34 includes a first end 341 and a second end 342 connected to the second handle 42. One end is fixedly connected to the second handle 42 (also referred to as the fixed end), and the other end is movably connected to the second handle 42 (the movable end), or both ends may be movably connected. The movable connection may be, for example, connection to the second base, with the second drive sleeve threadedly driving the second base to slide so as to loosen or tighten the control wire 34.

Regarding the control wire 34 as a whole, its distal portion is threaded around the artificial implant 100. The threading may pass through structural gaps on the proximal side of the artificial implant 100 or through dedicated connecting holes. The artificial implant 100 is controlled by loosening or tightening the control wire 34.

After the artificial implant 100 is deployed, it needs to be separated from the control wire 34. This can be achieved by disengaging the fixed end and/or the movable end of the control wire 34 from the second handle 42. For example, the movable end may be clamped and fixed to the second base; when release is required, the clamp is released so that the movable end becomes free and can be withdrawn from the artificial implant 100. Alternatively, the fixed end may be connected to the second handle 42 by clamping or fasteners, and when release is required, the fixed end is correspondingly disengaged.

In other embodiments, the control wire 34 may be severed by cutting or other means to achieve separation from the artificial implant 100. For example, a wire cutting mechanism may be provided within the second handle 42, or a separate wire cutting instrument may be used to cut the wire. Of course, partial melting may also be used to sever the wire.

Regarding the severing location of the control wire 34, the severing may occur at the proximal side, for example near the fixed end and/or the movable end, or at the distal side, for example, a wire cutting instrument may extend to the distal end near the connection point between the control wire 34 and the artificial implant 100.

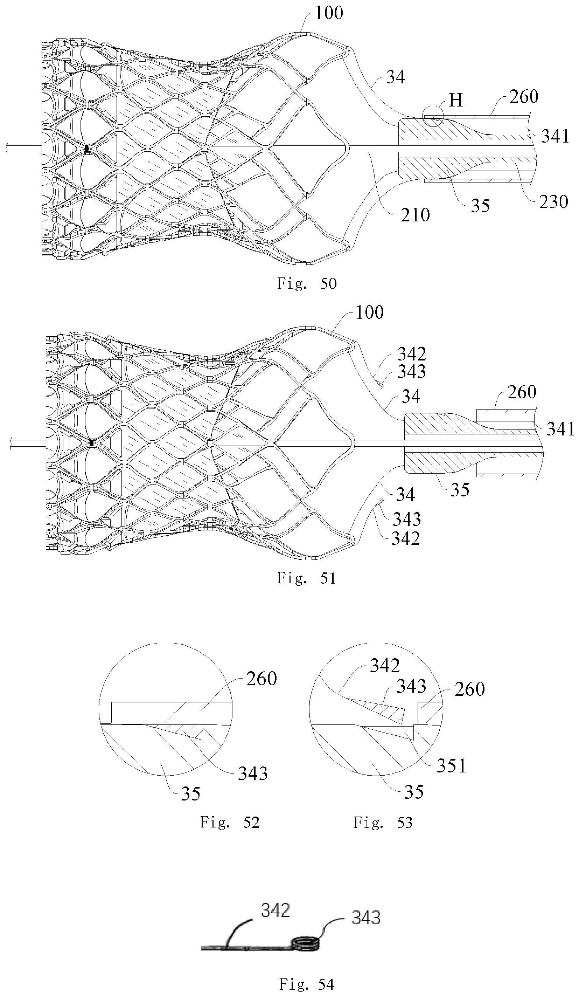

As shown in FIGS. 50 to 53, in another embodiment, the inner shaft assembly comprises an inner core 210 and an inner sheath tube 230. A lock base 35 is connected to a distal portion of the inner sheath tube 230. The proximal end of the inner sheath tube is fixed to the handle, i.e., fixed relative to the handle. One end of the inner core 210 is an extension segment extending out of the distal end of the inner sheath tube 230, and the other end may be fixed to the lock base 35 or extend through the lock base 35. The first end 341 of the control wire 34 serves as a movable end and is connected to the second handle. The second end 342, located at the distal end, passes sequentially through the lock base 35 and the artificial implant 100 and then extends proximally to connect to the lock base 35. For example, a barb may be provided on the wall of the lock base 35 for connecting the second end 342.

Similarly, the movable end may be connected to the third base and driven by the threaded engagement of the second drive sleeve. The anti-detachment structure at the distal end may utilize its own shape change or cooperate with other components to restrict or release the connection with the artificial implant 100.

For example, the catheter assembly may further include a retaining member 260 for limiting the second end 342 from detaching from the artificial implant 100.

The retaining member 260 acts on the second end 342 to keep the artificial implant under the action of the control wire, further limiting the second end from detaching from the lock base. The second end 342 has a movement path that allows it to switch to a separated state. The retaining member 260 has:
a first position, in which it prevents the second end 342 from switching to the separated state;
a second position, in which it allows the second end 342 to switch to the separated state.

The retaining member 260 slides axially. For example, the retaining member 260 is tubular and slidably sleeved on the outer periphery of the inner sheath tube 230. The proximal end of the retaining member 260 is connected to a base of the second handle (e.g., the second base).

The second end of the control wire 34 is provided with an anti-detachment structure 343 to prevent unintended detachment from the artificial implant 100.

In the locked state, the anti-detachment structure 343 and the lock base 35 are in an engaged state, and a disengaged state where the connection is released.

The anti-detachment structure 343 may be an enlarged end, such as a coiled or helical structure as shown in FIG. 54. An engagement groove 351 may also be provided on the outer periphery of the lock base 35. In the engaged state, the anti-detachment structure 343 is placed into the engagement groove 351 and wrapped by the retaining member 260 (as shown in FIG. 52). In the disengaged state, the anti-detachment structure is removed from the engagement groove (as shown in FIG. 53).

The control wire 34 may be a single wire or multiple wires. The retaining member 260 may be configured separately for each control wire 34, or all control wires 34 may share the same retaining member 260.

In one embodiment, the anti-detachment structure is made of a deformable material, and the connection with the artificial implant 100 is restricted or released by shape change. For example, the anti-detachment structure may be a shape memory alloy, such as a Nitinol wire.

After the distal end of the control wire 34 passes through the artificial implant 100, the anti-detachment structure itself is bent or coiled and is in a first state to maintain the connection with the artificial implant 100. When detachment is required, the anti-detachment structure deforms, and the bent or coiled portion expands into a second state, allowing detachment from the artificial implant 100.

In one embodiment, the anti-detachment structure may be heat-set in the second state. The delivery system further comprises a retaining member that restricts the anti-detachment structure in the first state. The retaining member may act on the anti-detachment structure by clamping, compressing, or other means. When the retaining member is separated from the anti-detachment structure or the manner of cooperation is changed, the anti-detachment structure self-drives to return to the second state.

In one embodiment, the anti-detachment structure may be heat-set in the first state. The delivery system further comprises a driving member that urges the anti-detachment structure to switch to the second state. For example, the driving member may use an electrical signal to cause the anti-detachment structure to deform toward the second state.

In the delivery system of the present disclosure, the support rod serves as a connecting member between the second handle and the first handle, and also enhances the structural strength of the control handle. It cooperates with the sliding locking mechanism to maintain the length dimension of the control handle and meet operational requirements, such as rapid retraction of the artificial implant, and precise control of the artificial implant during retrieval.

The technical features of the above embodiments can be combined in any manner. For the sake of brevity, not all possible combinations of the technical features in the above embodiments are described. However, as long as there is no contradiction in the combination of these technical features, they should be considered within the scope of this specification. When technical features from different embodiments are shown in the same drawing, that drawing shall be deemed to also disclose the combined examples of the various embodiments involved.

The above embodiments are merely several implementations of the present disclosure, and their descriptions are relatively specific and detailed, but they should not be construed as limiting the scope of the patent disclosure. It should be noted that for those of ordinary skill in the art, several variations and improvements can be made without departing from the concept of the present disclosure, and such variations and improvements shall all fall within the protection scope of the present disclosure.

## Claims

1. A delivery system for an artificial implant having opposite distal and proximal ends, the delivery system comprising a control handle and a catheter assembly having a proximal end connected to the control handle, **characterized in that:**
the control handle comprises a first handle and a second handle cooperating with each other;
the catheter assembly comprises an outer sheath and an inner shaft assembly located within the outer sheath, the artificial implant being connected to a distal end of the inner shaft assembly and being controlled by the control handle, the first handle being configured to drive axial movement of the outer sheath to effect deployment and collapse of the artificial implant; the second handle being configured to control release of the artificial implant to detach the artificial implant from the delivery system.

2. The delivery system for an artificial implant according to claim 1, **characterized in that** each handle comprises a support member and a drive mechanism disposed on a respective support member;
the support member of the first handle comprises a first support body and a support rod fixed to the first support body and extending proximally; the support member of the second handle comprises a second support body slidably mounted on the support rod, a sliding locking mechanism being further provided on a distal side of the second support body, the sliding locking mechanism interacting with the support rod to maintain a relative position with respect to the first support body.

3. The delivery system for an artificial implant according to claim 2, **characterized in that** the support rod is provided with a limiting member, and the sliding locking mechanism cooperates with the limiting member to maintain the relative position of the second handle with respect to the first support body.

4. The delivery system for an artificial implant according to claim 3, **characterized in that** the limiting member is a positioning tooth, and a plurality of positioning teeth are arranged at intervals along the axial direction.

5. The delivery system for an artificial implant according to claim 3, **characterized in that** the sliding locking mechanism comprises:
a locking member movably mounted on the second support body, the locking member having a locked position engaged with the limiting member and an unlocked position disengaged from the limiting member;
an elastic member acting between the locking member and the second support body, biasing the locking member toward the locked position; and an operating button operatively coupled to the locking member, driving the locking member toward the unlocked position.

6. The delivery system for an artificial implant according to claim 5, **characterized in that** the locking member is provided with a mounting hole and is slidably sleeved on the support rod through the mounting hole, a clearance being provided between the mounting hole and the support rod in a direction perpendicular to a sliding direction of the support rod, and the locking member moving along the clearance when moving between the locked and unlocked positions.

7. The delivery system for an artificial implant according to claim 6, **characterized in that** a side wall of the mounting hole faces the limiting member, and the side wall is provided with an engaging portion that cooperates with the limiting member.

8. The delivery system for an artificial implant according to claim 5, **characterized in that** the support rod comprises two support rods extending along the axial direction of the control handle and arranged side by side, and the sliding locking mechanism comprises two sliding locking mechanisms each acting on a respective support rod;
an abutment rib is fixed within the second support body, the elastic member being compressed and acting between the abutment rib and the locking member, and two abutment ribs being spaced apart from each other with an installation channel extending through spacing formed therebetween; and the locking member is provided with a positioning seat on a side facing the elastic member, the elastic member being inserted into or sleeved onto the positioning seat.

9. The delivery system for an artificial implant according to claim 2, **characterized in that** the support rod comprises two support rods arranged side by side and extending along an axial direction of the control handle.

10. The delivery system for an artificial implant according to claim 9, **characterized in that** the sliding locking mechanism comprises two sliding locking mechanisms, each acting on a respective support rod;
the sliding locking mechanism is provided with an operating button for unlocking, and two operating buttons are aligned with each other along a radial direction of the second handle and are arranged in opposite directions.

11. The delivery system for an artificial implant according to claim 1, **characterized in that** the delivery system is connected to a distal end of the artificial implant via a wire to achieve folding or compression of the artificial implant.

12. Delivery system for an artificial implant according to claim 11, **characterized in that** a wire control mechanism is provided on the second handle or at the distal end of the catheter assembly, the wire control mechanism comprising a mechanism for clamping the wire and/or a mechanism for releasing the wire.

13. The delivery system for an artificial implant according to claim 1, **characterized in that** the inner shaft assembly comprises two shafts, each having a proximal end connected to the second handle; the inner shaft assembly as a whole being movable relative to the first handle.

14. The delivery system for an artificial implant according to claim 1, **characterized in that** the inner shaft assembly comprises a first shaft, a second shaft and a third shaft, each having a proximal end connected to the second handle; the third shaft being fixedly connected to the second handle, and the first shaft and the second shaft being movably connected to the second handle.

15. The delivery system for an artificial implant according to claim 13 or 14, **characterized in that** one of the shafts of the inner shaft assembly is a steerable shaft or a steering wire.

16. The delivery system for an artificial implant according to claim 1, **characterized in that** the inner shaft assembly comprises a first shaft, a second shaft and a third shaft each having a proximal end connected to the second handle;
a lock base is connected to a distal end of the third shaft, the lock base being provided with a locking hole or a groove;
a locking wire is connected to a distal end of the second shaft, the locking wire being configured to be threaded through the artificial implant and then connected to the lock base to constrain or release the artificial implant;
a locking rod or a circumferentially arranged protruding structure is connected to a distal end of the first shaft, for maintaining connection between the locking wire and the lock base.
